# EUROPEAN PATENT APPLICATION

(11) **EP 4 212 875 A1**
(43) Date of publication of application: **19.07.2023**
(21) Application number: 21864469.8
(22) Date of filing: 06.09.2021
(51) Int. Cl.: G01N 33/53, G01N 33/543

(54) **BIOCHIP, METHOD FOR PRODUCING SAME, AND USE OF SAME**

(30) Priority: 07.09.2020 JP 2020150138
(71) Applicant: MITSUBISHI GAS CHEMICAL COMPANY, INC., Chiyoda-ku Tokyo 100-8324 (JP)
(72) Inventor: SAWANO Erika, Tokyo 100-8324 (JP); TAKASE Yuki, Tokyo 100-8324 (JP); SAKAI Haruka, Tokyo 125-8601 (JP); TASHIRO Hideo, Tokyo 113-0024 (JP); TASHIRO Tomoko, Tokyo 113-0024 (JP)
(74) Representative: Müller-Boré & Partner Patentanwälte PartG mbB
(86) International application number: PCT/JP2021/032732
(87) International publication number: WO 2022/050418

(57) **Abstract**

The present invention relates to a biochip having a substrate that has disposed on a surface at least one spot that contains a complex between a solid carrier that has been modified by a reactive group and a substance to be immobilized that has been modified via a spacer by a group that reacts with the reactive group. The present invention further relates, inter alia, to a method for producing the biochip and to a method for detecting a target substance using the biochip.

## Description

### Technical Field

The present invention relates to a biochip used in the detection or analysis of a biochemical reaction, etc., utilizing a biological material or the like immobilized on a substrate, a method of producing the biochip, a use thereof, etc.

### Background Art

In recent years, a biochip, in which a biological material such as an antibody, an antigen or a nucleic acid is immobilized on a chip, has been used in clinical tests and the like. In these biochips, biological materials such as proteins or nucleic acids that are used as materials to be immobilized are immobilized, in the form of spots, on the surface of a substrate. As methods of immobilizing a protein or the like on a substrate, the methods described in Patent Literatures 1 and 2 have been known.

### Citation List

### Patent Literature

Patent Literature 1: WO2018/154814
Patent Literature 2: WO2019/050017

### Summary of Invention

### Technical Problem

It has been desired to develop a biochip having high detection accuracy and high detection sensitivity.

### Solution to Problem

In one aspect, the present invention is, for example, as follows.
[1] A biochip having a substrate, wherein one or more spots are disposed on a surface of the substrate, each spot comprising a complex of a solid carrier modified with a reactive group and a material to be immobilized modified with a group reacting with the reactive group via a spacer.
[2] The biochip according to the above [1], wherein the reactive group is selected from the group consisting of avidin or a derivative thereof, biotin or a derivative thereof, alkyne, and azide.
[3] The biochip according to the above [1] or [2], wherein two or more spots are disposed, and the volume variation coefficient of the complex comprised in a single spot is less than 11.8%.
[4] The biochip according to any one of the above [1] to [3], wherein a surface treatment with a blocking agent is performed on the substrate.
[5] A method of producing a biochip, comprising spotting on a substrate a complex of a solid carrier modified with a reactive group and a material to be immobilized modified with a group reacting with the reactive group via a spacer, and immobilizing the spotted complex on the substrate.
[6] The method of producing a biochip according to the above [5], wherein spotting the complex on the substrate is carried out by using a non-contact dispenser comprising a nozzle with a diameter that is 100 to 2000 times greater than the average maximum diameter of the solid carrier.
[7] A material set for use in producing the biochip according to any one of the above [1] to [4], comprising a solid carrier modified with a reactive group, a material to be immobilized modified with a group reacting with the reactive group via a spacer, and a substrate.
[8] A method of detecting a target substance, comprising:
   providing the biochip according to the above [4], or allowing a blocking agent to act on the biochip according to any one of the above [1] to [3], in which a surface treatment with a blocking agent is not performed on the substrate;
   allowing a test sample possibly containing a target substance interacting with the material to be immobilized to come into contact with the complex in the biochip; and
   allowing a detection reagent to act on the biochip.
[9] The detection method according to the above [8], wherein the reactive group is avidin or a derivative thereof, and the blocking agent is biotin or a derivative thereof.
[10] The detection method according to the above [8] or [9], wherein the target substance is an antibody, and the detection reagent comprises a monoclonal antibody specific to the antibody.
[11] The detection method according to any one of the above [8] to [10], wherein the target substance is an antibody, and the detection reagent comprises an HRP-labeled antibody specific to the antibody.
[12] A kit for use in carrying out the detection method according to any one of the above [8] to [11], wherein the kit comprises the biochip according to any one of the above [1] to [4], a detection reagent, and as necessary, a blocking agent.
[13] A system for use in carrying out the detection method according to any one of the above [8] to [11], wherein the system comprises the biochip according to any one of the above [1] to [4] and a measurement device for a detection reagent.

### Advantageous Effects of Invention

The present invention has one or two or more of the following effects, depending on the aspect thereof.
(1) The biochip has excellent detection accuracy.
(2) The interaction between a material to be immobilized and a target substance is hardly inhibited.
(2) There are small variations among spots, in terms of the amounts of materials to be immobilized.
(3) The biochip has an excellent S/N ratio.
(4) The biochip has excellent detection sensitivity.
(5) The biochip can suppress the non-specific adsorption of a target substance.

### Brief Description of Drawings

Figure 1 is a schematic view showing a complex of a solid carrier modified with avidin or a derivative thereof and a biological material biotinylated via a spacer.
Figure 2 is an explanatory view showing one aspect of the biochip of the present invention. Figure 2(a) is a plan view, and Figure 2(b) is a cross-sectional view formed by cutting Figure 2(a) with the line A-A'.
Figure 3 is an explanatory view showing another aspect of the biochip of the present invention. Figure 3(a) is a plan view, and Figure 3(b) is a cross-sectional view formed by cutting Figure 3(a) with the line A-A'.
Figure 4 is an explanatory view showing another aspect of the biochip of the present invention. Figure 4(a) is a plan view, and Figure 4(b) is a cross-sectional view formed by cutting Figure 4(a) with the line A-A'.
Figure 5 is an explanatory view showing another aspect of the biochip of the present invention. Figure 5(a) is a plan view, and Figure 5(b) is a cross-sectional view formed by cutting Figure 5(a) with the line A-A'.
Figure 6 is a schematic view showing Biotinylated Peptides 2 immobilized on a bead.
Figure 7 shows optical microscope images of representative spots of Biochip 1 (left) and Biochip 2 (right).
Figure 8 shows the planar images of representative spots of Biochip 1 (left) and Biochip 2 (right) and profile graphs based on 3D images, wherein the images are obtained using a laser microscope.
Figure 9 shows photograph views showing the luminescence intensity of each spot of Experimental Group 1 (left) and Experimental Group 2 (right).
Figure 10 shows photograph views showing the luminescence intensity of each spot of Experimental Group 2 (left) and Experimental Group 3 (right).
Figure 11 shows photograph views showing the luminescence intensity of each spot of Experimental Group 3 (left) and Experimental Group 4 (right).
Figure 12 shows photograph views showing the luminescence intensity of each spot of Experimental Group 4 (left) and Experimental Group 5 (right). The spots enclosed with the dotted lines indicate spots in which luminescence was not detected in Experimental Group 4 but the luminescence could be confirmed in Experimental Group 5. On the other hand, the spots enclosed with the solid lines indicate those having equivalent luminescence intensity in the Experimental Groups 4 and 5.
Figure 13 is a graph showing the luminescence intensity of representative spots of Experimental Group 4 (×) and Experimental Group 5 (●). The longitudinal axis indicates luminescence intensity. Regarding many spots in which luminescence was not detected in the Experimental Group 4, luminescence was detected in the Experimental Group 5.
Figure 14 shows photograph views showing the luminescence intensity of each spot of Experimental Group 5 (left) and Experimental Group 6 (right).
Figure 15 shows photograph views showing the luminescence intensity of each spot of Biochips A to F.

### Description of Embodiments

Hereinafter, the embodiments of the present invention will be described. It is to be noted that the below-described materials, features and the like are not intended to limit the present invention, but may modify the present invention in various ways within the range of the spirit of the present invention.

All technical terms and scientific terms used in the present description have the same meanings as those that are generally understood by those skilled in the art, unless otherwise specified. All patents, patent applications, and other publications (including online information), which are cited in the present description, are incorporated herein by reference in their entirety. Moreover, the present description includes the contents as disclosed in the description and/or drawings of Japanese patent application (Japanese Patent Application No. 2020-150138), which are priority documents of the present application filed on September 7, 2020.

### 1. Biochip

The biochip of the present invention has a substrate, wherein one or more spots are disposed on a surface of the substrate, each spot comprising a complex of a solid carrier modified with a reactive group and a material to be immobilized modified with a group reacting with the reactive group via a spacer.

The solid carrier is not particularly limited, as long as it can be modified with a reactive group. Examples of the solid carrier may include microparticle carriers such as organic microparticles and inorganic microparticles. The microparticle carriers may also be magnetic microparticles. Examples of the magnetic microparticles may include, but are not limited to, beads having a uniform particle diameter, in which magnetizable materials such as yFe₂O₃ and Fe₃O₄ are uniformly covered with polymers having hydrophilicity (e.g., water-soluble polymers such as glycidyl methacrylate). Examples of such beads as commercially available products may include Dynabeads manufactured by Thermo Fisher Scientific, FG Beads manufactured by TAMAGAWA SEIKI Co., Ltd., Sera-Mag Magnetic Beads manufactured by GE HealthCare, and Magnosphere manufactured by JSR Life Sciences.

The size of such a solid carrier is not particularly limited. In the case of a microparticle carrier, a carrier having an average particle diameter of, for example, 0.1 to 5.0 µm, 0.1 to 3.0 µm, or 0.2 to 1.0 µm, can be used. The shape of the solid carrier may be either a spherical shape, or a non-spherical shape including an ellipsoid or a polyhedron such as a cube. In the case of a non-spherical carrier, a carrier having an average maximum diameter of, for example 0.1 to 5.0 µm, 0.1 to 3.0 µm, or 0.2 to 1.0 µm, can be used.

As such a reactive group, any given known reactive group can be used. Examples of the reactive group may include, but are not limited to, avidin or a derivative thereof, biotin or a derivative thereof, alkyne, azide, epoxy, a hydroxyl group, an amino group, a carboxyl group, a succinimide group, a tosyl group, protein A, and protein G.

The derivative of avidin is not particularly limited, as long as it interacts with biotin. Examples of the derivative of avidin may include Streptavidin, NeutrAvidin, Bradavidin, and Tamavidin. The derivative of biotin is not particularly limited, as long as it interacts with avidin. The derivative of biotin may be, for example, Biocytin.

The spacer is not particularly limited, as long as it does not inhibit the interaction between a material to be immobilized (e.g. a peptide) and a substance binding thereto, and the interaction between a reactive group and a group reacting therewith, and is also non-cleavable at the use of the biochip of the present invention (e.g. a spacer that does not have a cleavable bond such as an ester bond or a disulfide bond). Examples of the spacer may include a carbon chain and polyethylene glycol (PEG). The length of such a spacer is not limited, and it may be, for example, 5 to 150 Å, 6 to 100 Å, 7 to 80 Å, 8 to 60 Å, 9 to 50 Å, etc. When the spacer is a carbon chain, it may have a length of, for example, C1-C20 linear alkyl, C3-C10 linear alkyl, C4-C8 linear alkyl, or in particular, C6 linear alkyl. When the spacer is PEG, it may have a length of, for example, a dimer to a 24-mer, a dimer to a dodecamer, a dimer to an octamer, or in particular, a dimer to a tetramer.

As a group reacting with the reactive group, any given known group can be used. Examples of such a group may include, but are not limited to, avidin or a derivative thereof, biotin or a derivative thereof, alkyne, azide, a phenolic OH group, a hydroxyl group, an amino group, a carboxyl group, a His tag, and an antibody.

The material to be immobilized is not particularly limited, as long as it is a material capable of interacting with a target substance that is a detection target of the biochip. Examples of the material to be immobilized may include a biological material, an environmental material, and a material capable of interacting with these materials.

Examples of such a biological material may include a peptide, a nucleic acid, a sugar chain, or a mixture or a complex (e.g. glycoprotein) of one or more types selected from the aforementioned materials. It is to be noted that the term "peptide" is used herein as a generic name for substances formed by the peptide bonds of two or more amino acids. The peptide includes an oligopeptide and a polypeptide, and the polypeptide includes a protein.

Specific examples of the preferred biological material may include, but are not limited to: food allergens having immunogenicity, such as eggs, cow milks, meats, fish, crustaceans, mollusks, grains, beans, nuts, fruits, vegetables, beer yeasts, and gelatin; non-food allergens, such as pollens, mites, and house dusts; and tumor markers for liver cancer and breast cancer. Among others, specific examples of the biological material may include: main components of cow milk allergens, such as αs1-casein, αs2-casein, β-casein, κ-casein, α-lactalbumin, or β-lactoglobulin; main components of egg white allergens, such as ovomucoid, ovalbumin, or conalbumin; gliadin as a main component of wheat allergen; main proteins of *soba,* namely, proteins having a molecular weight of 24 kDa and 76 kDa; and Ara h1 as a main protein of peanuts. In particular, it is desirable to use at least one type of allergen selected from casein sodium, α-casein, β-casein, κ-casein, α-lactalbumin, β-lactoglobulin, ovomucoid, ovalbumin, and conalbumin. The above-described biological material may also include antibodies reacting against various types of allergens, various types of biomarkers (e.g., a tumor marker, an infection marker, a genetic disease marker, an endocrine disease marker, an inflammation marker, a fatigue marker, a stress marker, and a nutrition marker), biological materials (antigens, antibodies, aptamers, lectins, polynucleotides, enzymes, substrates, etc.) reacting with these biomarkers, and antigens regarding pathogens, autoimmune diseases, etc.

Examples of the material capable of interacting with the biological material may include the above-described biological materials, and also, artificial materials such as an antibody mimetic, a peptide mimetic, a modified nucleic acid, and a nucleic acid mimetic. Examples of the antibody mimetic may include, but are not limited, a monobody (adnectin), an affibody, an affimer, an affitin, an anticalin, an atrimer, a finomer, an armadillo repeat protein, a Kunitz domain, a knottin, an avimer, DARPin, an alpha body, an O body, and a lipibody. Examples of the peptide mimetic may include, but are not limited to, peptide-like materials including unnatural amino acids. Examples of the modified nucleic acid may include, but are not limited to, a base-modified nucleic acid, a sugar-modified nucleic acid, and a backbone-modified nucleic acid. Examples of the nucleic acid mimetic may include, but are not limited to, PNA and LNA.

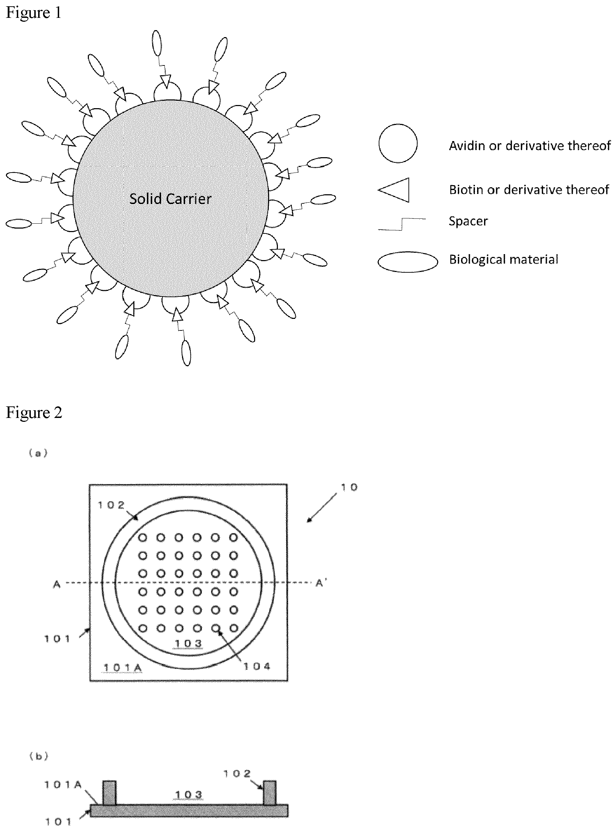

In a specific aspect, the above-described complex is composed of a solid carrier modified with avidin or a derivative thereof and a biological material biotinylated via a spacer. Figure 1 is a schematic view showing a complex of a solid carrier modified with avidin or a derivative thereof and a biological material biotinylated via a spacer. In the complex, the biological material is located farthest to the solid carrier, and the biological material is easily contacted with a target substance in a solution. Thus, compared with an aspect that does not include a spacer, the interaction between a biological material and a target substance is promoted.

The spot is typically a roughly circular region, and the diameter thereof may be fluctuated depending on the size of a biochip, the number of spots, etc. The diameter of a spot may be set to be, for example, 400 µm to 800 µm, and preferably 500 µm to 700 µm. The number of spots in a single biochip may be fluctuated depending on the size of a biochip, the size of a spot, etc. The number of spots is preferably 3 or more, more preferably 12 or more, and particularly preferably 18 or more. On the other hand, the number of spots is preferably 168 or less, and more preferably 144 or less. The range of the number of spots may be, for example, 1 to 200, 3 to 168, 18 to 144, or the like.

In one aspect, two or more spots are disposed on the biochip of the present invention, and the volume variation coefficient of the above-described complex comprised in a single spot is less than 11.8%. In a specific aspect, the above-described volume variation coefficient may be 11.5% or less, 11.0% or less, 10.5% or less, 10.0% or less, 9.5% or less, 9.0% or less, 8.5% or less, 8.0% or less, 7.5% or less, 7.0% or less, 6.5% or less, 6.0% or less, 5.5% or less, 5.0% or less, 4.5% or less, 4.0% or less, 3.5% or less, 3.0% or less, or the like. Moreover, the range of the above-described volume variation coefficient may be 0.5% to 3.0%, 0.5% to 3.5%, 0.5% to 4.0%, 0.5% to 4.5%, 0.5% to 5.0%, 0.5% to 5.5%, 0.5% to 6.0%, 0.5% to 6.5%, 0.5% to 7.0%, 0.5% to 7.5%, 0.5% to 8.0%, 0.5% to 8.5%, 0.5% to 9.0%, 0.5% to 9.5%, 0.5% to 10.0%, 0.5% to 10.5%, 0.5% to 11.0%, 0.5% to 11.5%, 1.0% to 3.0%, 1.0% to 3.5%, 1.0% to 4.0%, 1.0% to 4.5%, 1.0% to 5.0%, 1.0% to 5.5%, 1.0% to 6.0%, 1.0% to 6.5%, 1.0% to 7.0%, 1.0% to 7.5%, 1.0% to 8.0%, 1.0% to 8.5%, 1.0% to 9.0%, 1.0% to 9.5%, 1.0% to 10.0%, 1.0% to 10.5%, 1.0% to 11.0%, 1.0% to 11.5%, 1.5% to 3.0%, 1.5% to 3.5%, 1.5% to 4.0%, 1.5% to 4.5%, 1.5% to 5.0%, 1.5% to 5.5%, 1.5% to 6.0%, 1.5% to 6.5%, 1.5% to 7.0%, 1.5% to 7.5%, 1.5% to 8.0%, 1.5% to 8.5%, 1.5% to 9.0%, 1.5% to 9.5%, 1.5% to 10.0%, 1.5% to 10.5%, 1.5% to 11.0%, 1.5% to 11.5%, 2.0% to 3.0%, 2.0% to 3.5%, 2.0% to 4.0%, 2.0% to 4.5%, 2.0% to 5.0%, 2.0% to 5.5%, 2.0% to 6.0%, 2.0% to 6.5%, 2.0% to 7.0%, 2.0% to 7.5%, 2.0% to 8.0%, 2.0% to 8.5%, 2.0% to 9.0%, 2.0% to 9.5%, 2.0% to 10.0%, 2.0% to 10.5%, 2.0% to 11.0%, 2.0% to 11.5%, or the like. As the variation coefficient decreases, it is preferable.

Since the volume of the complex per spot is roughly proportional to the volume of a solid content per spot, the volume variation coefficient of the complex per spot can be indicated with the volume variation coefficient of a solid content per spot. The volume of a solid content present in a spot can be measured, for example, by subjecting a 3D image obtained using a laser microscope (in particular, a shape analysis laser microscope, such as VK-X Series, manufactured by Keyence Corporation) to image analysis software (Multi-analysis Application, manufactured by Keyence Corporation, etc.). Such a low variation coefficient can be typically achieved by applying a solution comprising the above-described complex to a substrate, using a non-contact dispenser. As such a non-contact dispenser, there can be used a non-contact dispenser having a nozzle whose diameter is 100 to 2000 times, preferably 150 to 1000 times, more preferably 200 to 700 times, and particularly preferably 500 times greater than the average maximum diameter of the solid carrier.

The biochip substrate of the present invention is not particularly limited, as long as it does not excessively affect test samples or biochemical reactions. For example, a resin material, a glass material, or the like can be used. The type of the resin material is not limited, and for example, a thermosetting resin or a thermoplastic resin can be used. In particular, if a light transmissive resin material such as polypropylene, polycarbonate, acryl, polystyrene, polyethylene terephthalate, a cycloolefin polymer or a cycloolefin copolymer is used, favorable visible light transmittance can be ensured. The type of polypropylene is not limited, and for example, a random copolymer of homopolypropylene or polypropylene and polyethylene can be used. In addition, the acryl is not limited, and for example, a copolymer of methyl polymethacrylate or methyl methacrylate and a monomer such as methacrylic acid ester, acrylic acid ester or styrene can be used. Besides, the terms "transparence" and "light transmittance" are used in the present invention to mean that the average transmittance in the wavelength region of a detected light is 70% or more. If a light transmissive material is used in a visible light region (wavelength: 350 to 780 nm), the condition of a sample in the chip can be easily confirmed by visual observation, but is not limited thereto. Moreover, a light non-transmissive resin material can also be used. Examples of such a light non-transmissive resin material may include, but are not limited to, resin materials such as polypropylene, polycarbonate, acryl, polystyrene, polyethylene terephthalate, a cycloolefin polymer, and a cycloolefin copolymer, to which coloring agents for resins (e.g. masterbatch, etc.) are added. The light non-transmissive resin material preferably has high light blocking properties, and the color of the light non-transmissive resin material is preferably black. The thickness of the substrate is not particularly limited. Since the substrate desirably has a certain extent of non-deforming property in the production process thereof, the thickness of the substrate is preferably 0.3 mm to 3.0 mm, more preferably 0.5 mm to 1.5 mm, and particularly preferably 0.7 mm to 1.0 mm.

In some aspect, a hydrophobic resin material is used in the substrate. In some aspect, the resin material is formed from a water-insoluble polymer. In some aspect, the resin material does not comprise dextran, PEG, or a derivative thereof.

In the biochip of the present invention, a surface treatment with a blocking agent may be performed on the substrate. By such a surface treatment, non-specific adsorption of substances contained in a test sample on the biochip can be suppressed. The blocking agent is not particularly limited, as long as it can suppress non-specific adsorption of the material to be immobilized on the biochip. When the reactive group is avidin or a derivative thereof, a blocking agent comprising biotin or a derivative thereof is preferable. By a surface treatment with a blocking agent comprising biotin or a derivative thereof, an unreacted biotin-binding site of the avidin or a derivative thereof on the solid carrier is inactivated, and non-specific adsorption of the biological material contained in the test sample can be suppressed. The blocking agent may comprise albumin, skimmed milk, a MPC polymer, PEG, and the like, as well as biotin or a derivative thereof. Moreover, when the target substance is a protein-binding substance, for example, an antibody or the like, the blocking agent is preferably a protein-free blocking agent.

A hydrophilization treatment may also be performed on the above-described substrate. By performing such a hydrophilization treatment, non-specific adsorption on the substrate can be prevented, and the above-described complex can be strongly immobilized on the substrate. The term "immobilization" is used in the present description to mean that a strong chemical bond such as a covalent bond is formed between the above-described complex and the surface of the substrate, and thus, the immobilization in the present description is distinguished from a weak chemical bond (also referred to as chemical adsorption) such as a hydrogen bond, or physical adsorption due to Van der Waals force or the like. In addition, the term "non-specific adsorption" is used in the present description to mean that a material to be immobilized and/or a substance specifically reacting with the material to be immobilized are adsorbed on a region on the surface of a substrate, on which the above-described complex is not immobilized. When the material to be immobilized is, for example, an antigen, a specific example of the substance specifically reacting with the above-described complex may be an antibody. When the material to be immobilized is an antibody, a specific example of the substance specifically reacting with the above-described complex may be an antigen.

The hydrophilization treatment may be performed on one or several portions on the surface of the substrate, or may also be performed on the entire surface of the substrate. When the hydrophilization treatment is performed on a portion of the substrate surface, the treatment may be performed, for example, on the entire upper surface of the substrate, or on one or several portions on the upper surface of the substrate. The area, on which the hydrophilization treatment is performed, may be 10% or more, 20% or more, 30% or more, 40% or more, 50% or more, 60% or more, 70% or more, 80% or more, 90% or more, or the like, of the total surface area of the substrate or the total surface area of the upper surface of the substrate. Moreover, the portion, on which the hydrophilization treatment is performed, may have various shapes. For example, the portion, on which the hydrophilization treatment is performed, may have a shape such as a round shape, an oval shape, or a polygonal shape, and thereby, for example, it becomes possible to form a plurality of round-shape hydrophilic portions in the form of spots on the surface of the substrate.

The hydrophilization treatment is not particularly limited, but examples of the hydrophilization treatment may include: the coating of the surface with inorganic materials having hydrophilicity, such as silica, or surfactants; chemical hydrophilization treatments such as a plasma treatment, a UV-ozone treatment, a corona treatment, or a flame treatment; and the imparting of hydrophilicity by physically forming a fine nanostructure (WO2011/024947). When the substrate is made of a resin, it is more preferable to apply chemical hydrophilization treatments such as a plasma treatment, a UV-ozone treatment, a corona treatment, or a flame treatment, by which the chemical bonds of molecules on the surface of the resin can be cleaved, and functional groups having polarity, such as, for example, OH (hydroxyl groups), CO (carbonyl groups), COOH (carboxyl groups), methoxy groups, peroxide groups, or polar ether groups can be thereby generated depending on the type of the resin; and it is particularly preferable to apply a UV-ozone treatment. In addition in one aspect, the hydrophilization treatment performed in the present invention does not involve the formation of a coating layer with a polymer, in particular, with a water-soluble polymer. Accordingly, the substrate according to this aspect has a hydrophilic surface, but does not have a coating layer. Besides, the term "hydrophilicity" is used in the present invention to mean that the contact angle (0/2 method) measured by a static contact angle measurement method (droplet method) is 1° or more and less than 80°, preferably 5° or more and less than 75°, and more preferably 10° or more and less than 70°.

In one aspect, the substrate has a surface comprising a hydrophilic reaction area, and preferably, a resin-made surface comprising a hydrophilic reaction area. The reaction area means a region on the substrate, on which the above-described complex is immobilized and is allowed to react with a detection target substance. The reaction area may be either a portion of the surface of the substrate, or the entire surface of the substrate. When the reaction area is a portion of the surface of the substrate, the surface of the substrate other than the reaction area may be either a hydrophilic or hydrophobic surface. When the reaction area is a portion of the surface of the substrate, the reaction area may have various shapes such as, for example, a round, oval, or polygonal shape, or a combination thereof. The reaction area may be either continuous or discontinuous. For example, the reaction area may form a plurality of spots that are not contacted with one another. The reaction area may be surrounded by a boundary capable of retaining a liquid therein. Otherwise, the reaction area may be covered with functional groups having polarity generated as a result that bonds associated with carbon of the resin are cleaved and the cleaved sites bind with oxygen. Examples of such a functional group may include, but are not limited to, a hydroxyl group, a carbonyl group, a carboxyl group, a methoxy group, a peroxide group, and a polar ether group.

A coating layer for preventing non-specific adsorption and immobilizing the above-described complex on the substrate can be established on the substrate. By immobilizing the material on the substrate via such a coating layer, non-specific adsorption can be prevented and detection sensitivity can be enhanced. Furthermore, the amount of the material to be immobilized exposed on the uppermost surface is increased according to the method of immobilizing the above-described complex on the substrate via a coating layer, in comparison to the method comprising mixing a polymer, a material to be immobilized and a photocrosslinking agent, and then applying the obtained mixture onto the substrate. Accordingly, a substrate that is excellent in terms of SN ratio and detection sensitivity can be obtained according to the method of immobilizing the above-described complex on the substrate via a coating layer.

The above-described coating layer is not particularly limited, as long as it enables promotion of the immobilization of the above-described complex, and/or suppression of non-specific adsorption. The coating layer can be constituted, for example, with various polymers. Among such polymers, a water-soluble polymer is preferable. By using such a water-soluble polymer, the use of water or a non-aqueous solvent other than alcohol, which may degenerate the above-described complex (including a material to be immobilized comprised in the complex), can be avoided. Hence, in the present invention, in order to prevent degeneration of the above-described complex, a water-soluble polymer is preferably used. Moreover, the water-soluble polymer is advantageous in that it has an excellent effect of suppressing non-specific adsorption. Herein, the term "water-soluble" is used to mean that, for example, the solubility of the polymer in water (grams of the polymer dissolved in 100 g of water) is 5 or more.

The number average molecular weight of the above-described polymer is not particularly limited, and it is generally approximately 3,500,000 to 5,000,000. By setting the molecular weight of the polymer to be approximately 500 to several hundreds of thousands, the number of crosslinks between polymers can be moderately maintained, and the reaction of the above-described complex with a substance to be subjected to the reaction with the above-described complex (e.g., a photocrosslinking agent, etc.) can be promoted.

Examples of the water-soluble polymer may include bipolar polymers such as phosphorylcholine-containing polymers, and nonionic polymers. Examples of the bipolar polymers may include polymers comprising, as a main component, 2-methacryloyloxy phosphorylcholine (MPC) (e.g. "LIPIDURE^{®}" manufactured by NOF CORPORATION (LIPIDURE^{®}-CR2001, LIPIDURE^{®}-CM5206, etc.)). Examples of the nonionic polymers may include: polyalkylene glycol, such as PEG or polypropylene glycol; nonionic vinyl polymers comprising, as a constituent element(s), a single form of, or a mixture of monomer units, such as vinyl alcohol, methyl vinyl ether, vinyl pyrrolidone, vinyl oxazolidone, vinyl methyl oxazolidone, 2-vinyl pyridine, 4-vinyl pyridine, N-vinyl succinimide, N-vinyl formamide, N-vinyl-N-methyl formamide, N-vinyl acetamide, N-vinyl-N-methyl acetamide, 2-hydroxyethyl methacrylate, polyethylene glycol methacrylate, polyethylene glycol acrylate, acrylamide, methacrylamide, N,N-dimethyl acrylamide, N-isopropyl acrylamide, diacetone acrylamide, methylol acrylamide, acryloyl morpholine, acryloyl pyrrolidine, acryloyl piperidine, styrene, chloromethyl styrene, bromomethyl styrene, vinyl acetate, methyl methacrylate, butyl acrylate, methylcyano acrylate, ethylcyano acrylate, n-propylcyano acrylate, isopropylcyano acrylate, n-butylcyano acrylate, isobutylcyano acrylate, tert-butylcyano acrylate, glycidyl methacrylate, ethylvinyl ether, n-propylvinyl ether, isopropylvinyl ether, n-butylvinyl ether, isobutylvinyl ether, or tert-butyl vinyl ether; and natural polymers, such as gelatin, casein, collagen, gum Arabic, xanthan gum, tragacanth gum, guar gum, pullulan, pectin, sodium alginate, hyaluronic acid, chitosan, a chitin derivative, carrageenan, starches (carboxymethyl starch and aldehyde starch), dextrin, or cyclodextrin, and natural polymers including water-soluble cellulose derivatives, such as methyl cellulose, viscose, hydroxyethyl cellulose, hydroxyethylmethyl cellulose, carboxymethyl cellulose, or hydroxypropyl cellulose, but the examples of the water-soluble polymer are not limited thereto. Moreover, commercially available products of photocrosslinked-type water-soluble polymers formed on the basis of these polymers, for example, "BIOSURFINE-AWP" manufactured by Toyo Gosei Co., Ltd., which was produced on the basis of polyvinyl alcohol, or "LIPIDURE^{®}-CR2001" manufactured by NOF CORPORATION, which was produced on the basis of 2-methacryloyloxy phosphorylcholine (MPC), etc., can be used. Among these polymers, polyethylene glycol polymers (e.g. a vinyl polymer of polyethylene glycol (meth)acrylate), polymers comprising MPC as a main component (e.g. LIPIDURE^{®}-CR2001), and the like are preferable.

In order to enhance the adhesiveness between the coating layer and the substrate, a surface treatment can be performed on the substrate. The surface treatment is not particularly limited, and examples of the surface treatment may include the treatments of cleaving the chemical bonds of molecules on the resin surface of the substrate and generating hydrophilic functional groups such as OH (hydroxyl groups), CO (carbonyl groups) and COOH (carboxyl groups), methoxy groups, peroxide groups, or polar ether group depending on the type of the resin, including, for example, a plasma treatment, a UV-ozone treatment, a corona treatment, or a flame treatment.

The test sample, to which the biochip of the present invention can be applied, is not particularly limited. Examples of the test sample may include body fluids such as blood, plasma, serum, saliva, urine, lymph, cerebrospinal fluid, joint fluid, nasal discharge, ascites, aqueous humor and lacrimal fluid, sputum, and biopsy specimens, which become targets of allergy or tumor diagnosis, or the like.

Hereinafter, the illustrative embodiments of the biochip of the present invention will be described with reference to the drawings.

Figure 2(a) is a plan view showing one aspect of the biochip of the present invention (hereinafter also referred to as "Biochip (A)"). In Biochip (A) 10, ring-shaped Boundary 102 is formed on Upper Surface 101A of Substrate 101. The above-described Boundary 102 is formed into an annular shape having a predetermined width, and is protruded from Substrate 101 to a predetermined height, so as to form Reaction Area 103 for retaining a liquid in a space surrounded by Upper Surface 101A and Boundary 102 of Substrate 101. In Figure 1(a), a ring-shaped boundary is shown, but the shape of the boundary is not limited thereto, and various shapes such as oval and polygonal shapes (e.g., quadrangle, pentagon, hexagon, and octagon) may be adopted. In the above-described Reaction Area 103, Spot 104, on which a component reacting with the above-described test sample component (i.e., a material to be immobilized) is immobilized, is arranged. A plurality of the above-described Spots 104 can be arranged. A plurality of independent spots are arranged at predetermined positions with predetermined intervals.

Figure 2(b) is a cross-sectional view showing a cross-sectional structure obtained by cutting Biochip (A) 10 with the line A-A' (see Figure 1(a)). The lower limit of the height of Boundary 102 from Upper Surface 101A of Substrate 101 is not particularly limited, but it is preferably 1 mm or more, and more preferably 2 mm or more. The upper limit of the height of Boundary 102 from Upper Surface 101A of Substrate 101 is not particularly limited, either. Taking into consideration the intended use of the chip, it is preferably 10 mm or less. The width of Boundary 102 is not particularly, either. Taking into consideration structural stability and the size of the chip, it is preferably in the range of 0.5 mm to 5.0 mm, and more preferably in the range of 1.0 mm to 3.0 mm. The material of Boundary 102 is not particularly limited, as long as it does not affect the test sample or the biochemical reaction. The material can be appropriately selected from rubber compositions such as natural rubber, synthetic rubber, silicon rubber, fluorine rubber or urethane rubber, or resin materials that can be used as the above-described substrate.

On Spot 104, the above-described complex is immobilized. The number of Spots 104 or the arrangement thereof is not particularly limited, and any given number or arrangement can be applied. In terms of the simultaneous test of multiple items, the lower limit of the number of Spots 104 is preferably 3 or more, more preferably 12 or more, and particularly preferably 18 or more. From the viewpoint of the limited size of the reaction area and prevention of the overlapping of the detected signals with the signals of neighborhood spots upon detection, the upper limit of the number of Spots 104 is preferably 168 or less, and more preferably 144 or less.

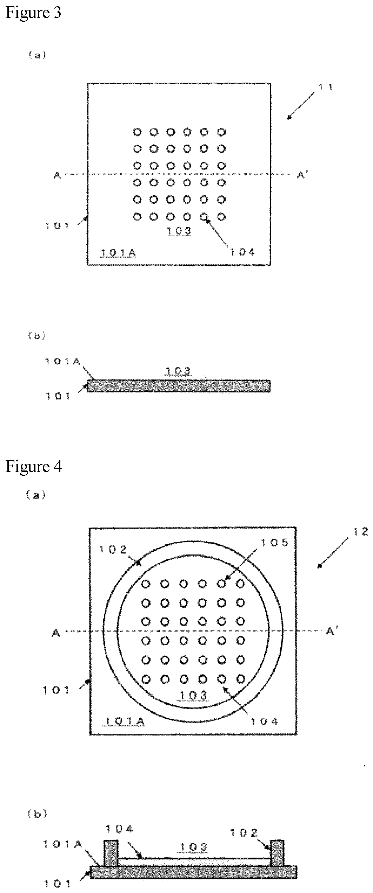

Figure 3(a) is a plan view showing another aspect of the biochip of the present invention (hereinafter also referred to as "Biochip (B)"). Biochip (B) 11 is different from Biochip (A) in that it does not have a boundary and the entire Upper Surface 101A of Substrate 101 serves as Reaction Area 103, but Biochip (B) 11 is identical to Biochip (A) in terms of other configurations. Since Biochip (B) does not need to establish a boundary, it is advantageous in terms of a simple production process and low production costs.

Figure 4(a) is a plan view showing one aspect of the biochip of the present invention (hereinafter also referred to as "Biochip (C)"). In Biochip (C) 12, ring-shaped Boundary 102 is formed on Upper Surface 101A of Substrate 101. The above-described Boundary 102 is formed into an annular shape having a predetermined width, and is protruded from Substrate 101 to a predetermined height, so as to form Reaction Area 103 for retaining a liquid in a space surrounded by Upper Surface 101A and Boundary 102 of Substrate 101. In Figure 3(a), a ring-shaped boundary is shown, but the shape of the boundary is not limited thereto, and various shapes such as oval and polygonal shapes (e.g., quadrangle, pentagon, hexagon, and octagon) may be adopted. In the above-described Reaction Area 103, Coating Layer 104 is formed to prevent non-specific adsorption and to immobilize biological materials on the substrate, and a plurality of Spots 105, on which components reacting with the above-described test sample components (i.e., the above-described complexes) have been immobilized, are arranged on the substrate via the Coating Layer 104. The above-described Spots 105 are arranged as a plurality of independent spots at predetermined positions with predetermined intervals.

Figure 4(b) is a cross-sectional view showing a cross-sectional structure obtained by cutting Biochip (C) 12 with the line A-A' (see Figure 3(a)). The lower limit of the height of Boundary 102 from Upper Surface 101A of Substrate 101 is not particularly limited, but it is preferably 1 mm or more, and more preferably 2 mm or more. The upper limit of the height of Boundary 102 from Upper Surface 101A of Substrate 101 is not particularly limited, either. Taking into consideration the intended use of the chip, it is preferably 10 mm or less. The width of Boundary 102 is not particularly, either. Taking into consideration structural stability and the size of the chip, it is preferably in the range of 0.5 mm to 5.0 mm, and more preferably in the range of 1.0 mm to 3.0 mm. The material of Boundary 102 is not particularly limited, as long as it does not affect the test sample or the biochemical reaction. The material can be appropriately selected from rubber compositions such as natural rubber, synthetic rubber, silicon rubber, fluorine rubber, or urethane rubber, or resin materials that can be used as the above-described substrate.

The thickness of Coating Layer 104 is not particularly limited, and any given thickness can be formed. The thickness is preferably 1 nm to 10 µm, more preferably 2 nm to 1 µm, and particularly preferably 10 nm to 100 nm.

On Spot 105, the above-described complex is immobilized. The number of Spots 105 or the arrangement thereof is not particularly limited, and any given number or arrangement can be applied. In terms of the simultaneous test of multiple items, the number of Spots 105 may be set as described above regarding the biochip of the present invention.

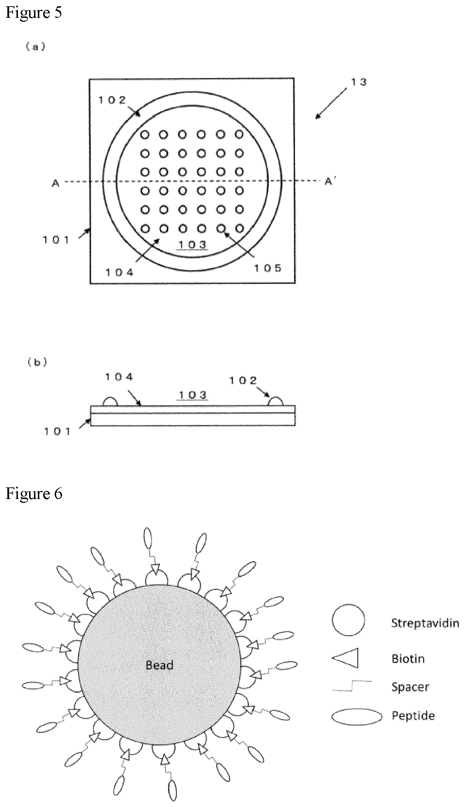

Figure 5(a) is a plan view showing another aspect of the biochip of the present invention (hereinafter also referred to as "Biochip (D)"). Biochip (D) 13 is different from Biochip (C) in that Coating Layer 104 for preventing non-specific adsorption and immobilizing biological materials is formed on the entire upper surface of Substrate 101. Accordingly, ring-shaped Boundary 102 is formed on Coating Layer 104. The above-described Boundary 102 is formed into an annular shape having a predetermined width, and is protruded from Coating Layer 104 to a predetermined height, so as to form Reaction Area 103 for retaining a liquid in a space surrounded by Coating Layer 104 and Boundary 102. The cross-section of Boundary 102 in this aspect is a semi-cylindrical form. However, the cross-sectional shape of Boundary 102 is not particularly limited, as long as a liquid can be retained in the space surrounded by Boundary 102. Thus, various shapes such as a triangle, a trapezoidal shape, or an L-shape can be adopted, as well as a rectangle as in the case of Biochip A. A plurality of Spots 105, on which components reacting with the above-described test sample components (i.e., materials to be immobilized) have been immobilized, are arranged on Coating Layer 104 of the above-described Reaction Area 103. The above-described Spots 105 are arranged as a plurality of independent spots at predetermined positions with predetermined intervals.

Figure 5(b) is a cross-sectional view showing a cross-sectional structure obtained by cutting Biochip (D) 13 with the line A-A' (see Figure 4(a)). The range of the height of Boundary 102 from Coating Layer 104 is the same as the range of the height of Boundary 102 from Upper Surface 101A of Substrate 101 in Biochip (C). Also, the width and material of Boundary 102, the thickness of Coating Layer 104, and Spots 105 are the same as those of Biochip (C).

### 2. Method of producing biochip

Another aspect of the present invention relates to a method of producing a biochip, comprising a step of spotting a complex of a solid carrier modified with a reactive group and a material to be immobilized modified with a group reacting with the reactive group via a spacer on a substrate, and a step of immobilizing the spotted complex on the substrate (hereinafter also referred to as "the production method of the present invention").

As such a solid carrier modified with a reactive group, a commercially available product may be used, or such a solid carrier may be newly produced according to any given known method. Examples of the commercially available solid carrier may include: FG beads^{®} and FF beads, manufactured by TAMAGAWA SEIKI Co., Ltd.; Streptavidin-Binding NanoLink/ MagnaLink Beads, manufactured by Solulink; Streptavidin Magnetic Beads, manufactured by New England Biolabs; Magnosphere and IMMUTEX, manufactured by JSR Life Sciences; and Streptavidin-Coated Microsphere, manufactured by Bangs Laboratories.

The solid carrier modified with a reactive group can be produced by any given known method. For example, production of the solid carrier modified with avidin or a derivative thereof can be specifically carried out, for example, by treating a solid carrier having an amino group on the surface thereof with a crosslinker (glutaraldehyde, etc.) to generate a chemically active functional group (an aldehyde group, etc.) on the surface of the solid carrier, then mixing the activated solid carrier with avidin or a derivative thereof, so that a covalent bond can be formed by the reaction of the active functional group (an aldehyde group, etc.) on the bead with a functional group (an amino group, etc.) on the avidin or a derivative thereof, and then allowing the reaction mixture to react with a quenching agent (glycine, etc.) to inactivate unreacted active functional groups. Otherwise, the surface of such a solid carrier may be biotinylated, and avidin or a derivative thereof may be then bound to the resulting solid carrier (Japanese Patent Publication (Kokai) No. 2009-300349 A).

The material to be immobilized modified with a group reacting with the reactive group via a spacer can be produced by any given known method. For example, in the case of a peptide biotinylated via a spacer, the peptide is allowed to react with a biotin derivative having, at one end of a spacer, a group reacting with a functional group comprised in the peptide (i.e. a primary amine (-NH₂), a sulfhydryl group (-SH), or a carboxyl group (-COOH), etc.), so that the peptide can be biotinylated. Specifically, for example, a biotin derivative having a reactive group such as N-hydroxysuccinimide (NHS) ester, sulfo NHS ester, or tetrafluorophenyl (TFP) ester can be used with respect to the primary amine; and a biotin derivative having a reactive group such as a maleimide group or an iodoacetyl group can be used with respect to the sulfhydryl group. Moreover, a biological material having a C-H or N-H site can be biotinylated according to a photochemical reaction with a biotin derivative having a photo reactive group (e.g. arylazide such as TFPA). Such biotin derivatives are commercially available, or can be synthesized by known methods.

The complex of a solid carrier modified with a reactive group and a material to be immobilized modified with a group reacting with the reactive group via a spacer can be produced by allowing the above-described reactive group to react with the above-described group reacting with the reactive group under conditions in which the reaction takes place, for a certain period of time. For instance, the complex of the solid carrier modified with avidin or a derivative thereof and the biological material biotinylated via a spacer can be produced by allowing both of them to react with each other under conditions in which an avidin/biotin interaction takes place (e.g. in PBS at 4°C), for a certain period of time (about 1 to 2 hours).

The spotting of the above-described complex onto the substrate can be achieved, for example, by applying a dispersed solution of the complex in a predetermined amount onto the substrate. For application of the dispersed solution onto the substrate, for example, a method of spotting the dispersed solution using a non-contact dispenser, a method of spotting the dispersed solution using a micropipette or the like, a spotting method involving a pin method, a spotting method involving a piezoelectric method, etc. can be used. The method of spotting the dispersed solution using a non-contact dispenser can apply a more precise amount of dispersed solution onto the substrate, than methods that need contact with the substrate, such as the spotting method involving a pin method, and thus, the spotting method using a non-contact dispenser is preferable. In a specific aspect, there can be used a non-contact dispenser having a nozzle whose diameter is 100 to 2000 times, preferably 150 to 1000 times, more preferably 200 to 700 times, and particularly preferably 500 times greater than the average maximum diameter of the solid carrier. For instance, when the average maximum diameter of the solid carrier is 200 nm, a non-contact dispenser having a nozzle whose diameter is about 20 to 400 µm, preferably 30 to 200 µm, more preferably 40 to 140 µm, and particularly preferably 100 µm, can be used.

The spotted complex can be immobilized on the substrate according to any given known method. In one aspect, immobilization of the complex on the substrate is carried out using a photocrosslinking agent. As such a crosslinking agent, a photocrosslinking agent having at least two photoreactive groups in a single molecule can be used. Examples of the photoreactive groups possessed by the photocrosslinking agent may include azide groups (-N₃), acetyl groups, benzoyl groups and diazirine groups. In particular, azide groups are preferable because when the azide groups are irradiated with light, nitrogen molecules are dissociated and nitrogen radicals are generated, and these nitrogen radicals can bind not only to functional groups such as amino groups or carboxyl groups, but can also bind to carbon atoms constituting an organic compound, and can form covalent bonds with almost all organic matters. The photocrosslinking agent having such azide groups may be, for example, diazidostilbene. The photocrosslinking agent is preferably a water-soluble agent. The "water solubility" of the photocrosslinking agent means that an aqueous solution comprising the photocrosslinking agent in a concentration of 0.5 mM or more, and preferably 2 mM or more, can be given.

In some aspect, production of the biochip of the present invention comprising a hydrophilic reaction area may comprise:
producing a biochip substrate by a step of performing a hydrophilization treatment on a substrate (hereinafter referred to as "Step 1-1 "); and then,
a step of spotting, at least, the above-described complex, a photocrosslinking agent having at least two photoreactive groups in a single molecule, and a thickener and/or a surfactant on the above-described biochip substrate, and then applying a light to the substrate (hereinafter referred to as "Step 1-2").

Hereafter, the biochip of the present invention comprising a hydrophilic reaction area, and the biochip of the present invention having a coating layer and a reaction area surrounded by a boundary capable of retaining a liquid therein, will be taken as examples, and individual constituent elements of the production methods thereof will be described.

### (1) Method of producing the biochip of the present invention comprising a hydrophilic reaction area

Production of the biochip of the present invention comprising a hydrophilic reaction area may comprise:
producing a biochip substrate by a step of performing a hydrophilization treatment on a substrate (hereinafter referred to as "Step 1-1 "); and then,
a step of spotting, at least, the above-described complex, a photocrosslinking agent having at least two photoreactive groups in a single molecule, and a thickener and/or a surfactant on the above-described biochip substrate, and then applying a light to the substrate (hereinafter referred to as "Step 1-2").

### Step 1-1

Step 1-1 may comprise a step of performing a hydrophilization treatment on a substrate. The hydrophilization treatment of the substrate is not particularly limited, as long as it is a treatment of hydrophilizing the surface of the substrate. Examples of the hydrophilization treatment may include: a method comprising applying silica, a surfactant and the like that are dissolved or suspended in a liquid onto a substrate according to spin-coating, coating, spraying, immersion, etc., and then drying it; a method of performing a chemical hydrophilization treatment such as a plasma treatment, a UV-ozone treatment, a corona treatment, or a flame treatment on a substrate; a method of transcribing a fine nanostructure on a substrate, or roughening a substrate with a liquid medicine to physically form a nanostructure on the substrate, so as to impart hydrophilicity to the substrate; and a method of coating a substrate with a hydrophilic polymer. A method of performing a chemical hydrophilization treatment such as a plasma treatment, a UV-ozone treatment, a corona treatment, or a flame treatment on a substrate, in which the chemical bonds of molecules on the resin surface are cleaved and thereby hydrophilic functional groups having polarity, such as OH (hydroxyl groups), CO (carbonyl groups), COOH (carboxyl groups), methoxy groups, peroxide groups, or polar ether groups can be generated depending on the type of the resin, is more preferable, and a method involving a UV-ozone treatment is particularly preferable.

A step of forming a boundary on a substrate to form a reaction area can be established. The method of forming a reaction area is not particularly limited. Examples of the method of forming a reaction area that can be used herein may include: a method of allowing a ring that has previously been formed with the above-described rubber composition or the above-described resin material to adhere to a substrate, using an adhesive or the like; and a method of subjecting a substrate constituted with the above-described resin material to various types of resin molding methods such as injection molding or vacuum forming, or to mechanical cutting or the like, so as to mold a ring. The adhesive used herein is not particularly limited, as long as it does not affect the test sample or the biochemical reaction. The adhesive can be appropriately selected from commercially available adhesives.

### Step 1-2

Step 1-2 may comprise a step of spotting, at least, the above-described complex, a photocrosslinking agent having at least two photoreactive groups in a single molecule, and a thickener and/or a surfactant, in the form of grid-like spots, on above-described biochip substrate, and a step of applying a light to the substrate.

### (2) Method of producing the biochip of the present invention having a coating layer and a reaction area

Production of the biochip of the present invention having a coating layer and a reaction area surrounded by a boundary capable of retaining a liquid therein may comprise:
producing a biochip substrate by a step selected from a step of establishing a coating layer for preventing non-specific adsorption and immobilizing biological materials on the substrate, and a step of forming a boundary on the substrate to form a reaction area (hereinafter referred to as "Step 2-1"); and then,
a step of spotting, at least, the above-described complex, a photocrosslinking agent having at least two photoreactive groups in a single molecule, and a thickener and/or a surfactant on the above-described biochip substrate, and then applying a light to the substrate (hereinafter referred to as "Step 2-2").

### Step 2-1

Step 2-1 may comprise a step selected from a step of establishing a coating layer for preventing non-specific adsorption and immobilizing biological materials on the substrate and a step of forming a boundary on the substrate to form a reaction area. As long as Step 2-1 comprises a step(s) selected from the above-described steps, the order of performing these steps may be changed. In addition, Step 2-1 may comprise a step of performing a surface treatment on the substrate, before the step of establishing a coating layer.

The method of performing a surface treatment in the step of performing a surface treatment on the substrate is not particularly limited, as long as the method can enhance the adhesiveness between the substrate and the coating layer. For example, a known surface modification method such as a plasma treatment, a UV-ozone treatment, or a corona treatment can be applied.

A coating layer for preventing non-specific adsorption on a substrate and immobilizing biological materials on the substrate can be formed according to a known method such as the spin-coating, coating or spraying of a coating solution comprising a water-soluble polymer, or immersion of the substrate in a coating solution. For example, the coating solution can be prepared by dissolving a water-soluble polymer in a solvent. As such solvents, water, a lower alcohol mixed with water at any given ratio, and a mixture thereof can be used. As such lower alcohols, methanol, ethanol, and isopropanol are preferable. Among others, it is preferable to use a mixed solvent of ethanol and water.

The concentration of the polymer in the above-described coating solution is not particularly limited. The polymer concentration can be set at, for example, 0.0001 to 10 parts by mass, and preferably 0.001 to 1 part by mass. The concentration of the photocrosslinking agent can be set at, for example, 1 to 20 parts by mass, and preferably 2 to 10 parts by mass, with respect to the above-described polymer.

The coating solution containing the above-described polymer preferably comprises a photocrosslinking agent having at least two photoreactive groups in a single molecule. In the present invention, the "photoreactive group" means a group that generates radicals as a result of light irradiation.

In the above-described photocrosslinking agent, photoreactive groups generate radicals as a result of light irradiation, so that covalent bonds can be formed with amino groups, carboxyl groups, carbon atoms constituting an organic compound, etc. Thereby, a coating solution comprising the above-described photocrosslinking agent is applied onto the substrate, followed by light irradiation, so that the substrate can be bound to the polymer via the photocrosslinking agent, and so that a polymer layer having a non-specific adsorption preventing effect can be formed on the substrate. Besides, in the present invention, without using the above-described photocrosslinking agent, or together with the above-described photocrosslinking agent, a photoreactive group and/or a group capable of covalently or coordinately binding to the substrate surface are introduced into the above-described polymer, so that the substrate can be bound to the polymer by utilizing the groups possessed by the polymer.

The water-soluble polymer and the photocrosslinking agent comprised in the coating layer of the present invention are known. The water-soluble polymer and the photocrosslinking agent can be produced by known production methods and also, are commercially available. The film thickness of the above-described coating layer is not particularly limited, but it is preferably 1 nm to 10 µm, more preferably 2 nm to 1 µm, and particularly preferably 10 nm to 100 nm.

In the present invention, as mentioned above, it is preferable to stabilize a coating layer by applying the coating layer to the substrate and then aging it under constant temperature and humidity conditions. The temperature is preferably 5°C to 40°C, more preferably 20°C to 30°C, and particularly preferably about 25°C. The humidity is preferably 40% to 80%, more preferably 50% to 70%, and particularly preferably about 60%. The aging period is preferably 1 day to 2 months, more preferably 3 days to 1 month, and particularly preferably 5 days to 2 weeks.

The method of forming a boundary on the substrate is not particularly limited. A method of adhering a ring that has previously been formed with the above-described rubber composition or the above-described resin material onto the substrate, using an adhesive or the like, or a method of molding a substrate constituted with the above-described resin material into a ring according to various types of resin molding methods such as injection molding or vacuum forming, mechanical cutting, etc., can be applied. The adhesive is not particularly limited, as long as it does not affect the test sample or the biochemical reaction. Thus, an adhesive can be appropriately selected from commercially available adhesives.

### Step 2-2

Step 2-2 may comprise: a step of spotting, at least, the above-described complex, a photocrosslinking agent having at least two photoreactive groups in a single molecule, and a thickener and/or a surfactant, in the form of grid-like spots, on the above-described biochip substrate; a step of applying a light to the substrate; and a step of removing unreacted components.

The above-described complex and the above-described photocrosslinking agent are preferably dispersed or dissolved in a solution. The solution is not particularly limited, and a known buffer can be used. Examples of the buffer composition may include a PBS buffer, a HEPES buffer, a Tris buffer, and a MES buffer. The use of a HEPES buffer is preferable because the above-described complex can be favorably dispersed in the HEPES buffer. The solution, in which the above-described complex and the photocrosslinking agent are dispersed or dissolved, may be referred to as a stamp solution at times.

The concentration of the above-described material to be immobilized and the concentration of the above-described complex are not particularly limited. The material to be immobilized is allowed to react, preferably in a concentration of 0.5 mg/mL to 50 mg/mL, and more preferably in a concentration of 1 mg/mL to 25 mg/mL with the solid carrier. The above-described complex is used preferably in a concentration of 1 mg/mL to 10 mg/mL, and more preferably in a concentration of 2.5 mg/mL to 7.5 mg/mL.

The concentration of the above-described photocrosslinking agent is not particularly limited, but it is preferably 0.01 mg/mL to 1 g/mL, and more preferably 0.2 mg/mL to 0.2 g/mL.

The solution, in which the above-described complex and the above-described photocrosslinking agent are dispersed or dissolved, preferably may further comprise a thickener and/or a surfactant. By allowing the above-described solution to comprise a thickener, the size of a spot can be controlled when the solution is spotted on the substrate. More specifically, when a solution having a predetermined volume is spotted on a substrate, as the concentration of a thickener in the solution increases, the size of a spot (the area of a spot contacted with the substrate) tends to decrease. Moreover, by allowing the solution to comprise a surfactant, a phenomenon, whereby the above-described complex accumulates in the gas-liquid interface and the above-described complex is thereby localized in the margin of the spot, can be prevented. Also, such a surfactant contributes to the improvement of the affinity for the substrate. When a solution having a predetermined volume is spotted on a substrate, as the concentration of a surfactant in the solution increases, the size of a spot (the area of a spot contacted with the substrate) tends to increase. Accordingly, a stamp with any given size can be formed by controlling the concentration of the thickener and the surfactant.

The above-described thickener is not particularly limited, as long as it does not affect the test sample or the biochemical reaction. A commercially available thickener can be used. Examples of the thickener may include celluloses and derivatives thereof, polysaccharides, vinyl compounds, vinylidene compounds, polyglycol compounds, polyvinyl alcohol compounds, and polyalkylene oxide compounds. Specific examples of the thickener that can be used herein may include gellan gum, xanthan gum, curdlan, pullulan, a guar gum derivative, locust bean gum, carrageenan, pectin, β glucan, tamarind gum, psyllium seed gum, dextran, glycerin, carboxymethyl cellulose, hydroxyethyl cellulose, hydroxymethylpropyl cellulose, lanolin, methyl cellulose, Vaseline, PEG, polyvinyl alcohol, polyvinyl pyrrolidone, a carboxyl vinyl polymer, polyvinyl pyrrolidone, polyvinyl alcohol, a dextrin acid fatty ester, and an inulin acid fatty ester. It is preferable to use one or more selected from hydroxyethyl cellulose, methyl cellulose, polyvinyl pyrrolidone, and polyvinyl alcohol, and it is more preferable to use polyvinyl alcohol.

The concentration of the above-described thickener is not particularly limited. For example, when polyvinyl alcohol is used as a thickener, it is used in a concentration of preferably 0.01 part by weight to 1 part by weight, more preferably 0.02 parts by weight to 0.5 parts by weight, and particularly preferably 0.03 parts by weight to 0.3 parts by weight, with respect to 100 parts by weight of the above-described solution.

The above-described surfactant is not particularly limited, as long as it does not affect the test sample or the biochemical reaction. A commercially available nonionic surfactant can be used. Examples of the surfactant that can be used herein may include polyoxyethylene(10) octylphenyl ether [Triton X-100], polyoxyethylene(8) octylphenyl ether [Triton X-114], polyoxyethylene sorbitan monolaurate [Tween 20], polyoxyethylene sorbitan monooleate [Tween 80], polyoxyethylene(23) lauryl ether [Brij35], polyoxyethylene(20) lauryl ether [Brij 58], Pluronic F-68, PEG, and a mixture of two or more types thereof. It is preferable to use one or more types selected from Triton X-100, Tween 20, and Tween 80, and it is more preferable to use Tween 20.

The concentration of the above-described surfactant is not particularly limited. For example, when Tween 20 is used as a surfactant, it is used in a concentration of preferably 0.001 part by weight to 1 part by weight, more preferably 0.005 parts by weight to 0.5 parts by weight, and particularly preferably 0.01 part by weight to 0.3 parts by weight, with respect to 100 parts by weight of the above-described solution.

The viscosity of the stamp solution is not particularly limited, as long as a spot having a desired shape can be formed. The viscosity of the stamp solution at 25°C may be, for example, 0.4 mPa·s to 40 mPa·s, preferably 0.5 mPa·s to 10 mPa·s, more preferably 0.6 mPa·s to 4 mPa·s, particularly preferably 0.8 mPa·s to 2 mPa·s, and further particularly, approximately 1.3 mPa·s. On the other hand, the viscosity of the stamp solution at 25°C may be 0.6 mPa·s to 10 mPa·s, 0.8 mPa·s to 4 mPa·s, 1.0 mPa·s to 2 mPa·s, 1.12 mPa·s to 2 mPa·s, 1.13 mPa·s to 2 mPa·s, 1.14 mPa·s to 2 mPa·s, 1.15 mPa·s to 2 mPa·s, 1.16 mPa·s to 2 mPa·s, 1.17 mPa·s to 2 mPa·s, 1.18 mPa·s to 2 mPa·s, 1.19 mPa·s to 2 mPa·s, 1.2 mPa·s to 2 mPa·s, or the like.

The method of spotting the above-described complex and photocrosslinking agent onto the substrate is not particularly limited, and for example, the method as described above regarding the production method of the present invention can be applied. The diameter of such a spot can be determined as described above regarding the biochip of the present invention.

Immobilization of the above-described complex can be carried out by applying a light to a substrate, after application of the complex to the substrate, preferably after the drying of the spotted solution. The light is not particularly limited, as long as the used photoreactive groups can generate radicals under the light. In particular, when azide groups are used as such photoreactive groups, ultraviolet rays (for example, with a wavelength of 10 to 400 nm) are preferable. The irradiation time can be set at, for example, 10 seconds to 120 minutes, preferably 30 seconds to 60 minutes, and more preferably 1 minute to 30 minutes. Since the complex is promptly immobilized by light irradiation, the irradiation time is almost equal to the time required for immobilization. The dose of the irradiated light is not particularly limited, but it is generally approximately 1 mW to 100 mW per cm². The photoreactive groups contained in the photocrosslinking agent (or when the polymer has photoreactive groups, the photoreactive groups contained in the polymer) generate radicals as a result of the light irradiation, so that the polymer layer can be bound to the above-described complex via the photocrosslinking agent.

After a desired material has been immobilized on a substrate as described above, the substrate is washed according to a known method, so that unreacted components and the like can be removed. However, such washing is not essential, and the immobilized material may be subjected to productization, while the stamp solution remains. Thus, a biochip, on which a desired material to be immobilized has been immobilized, can be obtained. When the washing is not carried out, the immobilized complex, as well as stamp solution components such as a thickener and/or a surfactant, may adhere onto the spot on the biochip. Such a stamp solution residue can be removed by washing the biochip, as appropriate, before the use thereof.

### 3. Material set for use in producing biochip

Another aspect of the present invention relates to a material set for use in producing the biochip of the present invention, comprising a solid carrier modified with a reactive group, a material to be immobilized modified with a group capable of reacting with the reactive group via a spacer, and a substrate.

Individual constituent elements of the material set of the present invention are as described above regarding the biochip of the present invention. The material set of the present invention may comprise the above-described constituent elements, and also, materials for a stamp solution used upon the spotting of the complex onto the substrate (e.g. a crosslinking agent for immobilizing the complex on the substrate (for example, the above-described photocrosslinking agent), a thickener, a surfactant, etc.), and instructions for producing the biochip of the present invention, such as, for example, -, information of sites including information regarding use methods (e.g. URL and 2D code), and media that record information regarding use methods (e.g. a flexible disc, CD, DVD, a Blu-ray disc, a memory card, or an USB memory). By using the material set of the present invention, a biochip having a desired layout can be produced.

### 5. Method of detecting target substance using biochip

Another aspect of the present invention relates to a method of detecting a target substance using a biochip.

One aspect of the detection method of the present invention relates to:
a method of detecting a target substance, comprising:
(1a) a step of providing the biochip of the present invention, in which a surface treatment with a blocking agent is performed on the substrate, or (1b) a step of allowing a blocking agent to act on the biochip of the present invention, in which a surface treatment with a blocking agent is not performed on the substrate;
(2) a step of allowing a test sample possibly containing a target substance interacting with the material to be immobilized to come into contact with the complex in the biochip; and
(3) a step of allowing a detection reagent to act on the biochip.

The step of allowing a blocking agent to act on the biochip of the present invention is not limited, and for example, this step can be achieved by applying blocking agents to spots disposed on the biochip, so that they are reacted for a predetermined period of time, and then washing the spots to remove unreacted blocking agents. The blocking agent is as described above regarding the biochip of the present invention, and in an aspect of using avidin or a derivative thereof as a reactive group, the blocking agent may comprise biotin.

The target substance is not particularly limited, as long as it can interact with the material to be immobilized that binds to the complex. Examples of the target substance may include proteins such as antibodies, antigens, enzymes, hormones or cytokines, nucleic acid molecules such as RNA or DNA, and sugar chains.

The test sample is typically a sample possibly containing a target substance. Examples of the test sample may include, but are not limited to, body fluids such as blood, plasma, serum, saliva, urine, lymph, cerebrospinal fluid, joint fluid, nasal discharge, ascites, aqueous humor and lacrimal fluid, sputum, and biopsy specimen. For instance, when the target substance is an antibody, blood, plasma, serum or the like can be used as a test sample.

The step of allowing a test sample to come into contact with the complex can be achieved, for example, by applying test samples to spots disposed on the biochip, then reacting them for a predetermined period of time (e.g. several minutes to several hours), and then washing the spots to remove unreacted target substances or the like. The test sample may be diluted and then used, as necessary.

The detection agent to be acted on the biochip is not particularly limited, as long as it can detect a target substance that binds to a material to be immobilized. The detection agent may be, for example, a substance that has a detectable label and reacts with a target substance. The substance reacting with a target substance is not limited, and any given substance known to bind to a target substance can be used. For example, when the target substance is a protein, an antibody specific to the protein and a ligand of the protein are included in the substance reacting with the target substance. When the target substance is a nucleic acid molecule, a nucleic acid molecule hybridizing with the nucleic acid molecule is included in the substance reacting with the target substance. The label added to the substance reacting with the target substance is not particularly limited, as long as it can be detected. Examples of the label may include a fluorescent material, a luminescent material, an enzyme, and a radioisotope.

Detection of the target substance may be different depending on the detection reagent used. For example, when a labeled substance that binds to the target substance is used as a detection reagent, the target substance can be detected by detecting the label that binds to the target substance. The label can be detected by various types of known methods that depend on the label. For example, when the label is an enzyme, a luminescent material or a coloring material that reacts with the enzyme is allowed to act on the enzyme, and luminescence intensity or absorbance is then measured, so that the label can be detected. Such detection may be carried out, either qualitatively or quantitatively.

In some aspect, a labeled monoclonal antibody specific to the target substance can be used as a detection reagent. By using such a monoclonal antibody, the binding of the antibody to an undesired site can be suppressed and the SN ratio can be enhanced upon the detection, when compared with a polyclonal antibody.

In some aspect, an HRP-labeled substance, for example, an HRP-labeled antibody can be used as a detection reagent. By using HRP as a label, for example, luminescence intensity higher than ALP can be obtained, and thereby, a sample having a high dilution rate can be used. As a result, the influence of contaminants contained in the sample can be reduced, and it becomes possible to improve the SN ratio.

In a specific aspect, an HRP-labeled monoclonal antibody specific to the target substance can be used as a detection reagent.

When the biochip is not subjected to a washing step and a blocking treatment, after immobilization of the complex, and when the components of the stamp solution remain in the substrate, the detection method of the present invention may also comprise a step of washing the biochip before the step (1b).

Another aspect of the detection method of the present invention is a method of detecting a target substance, comprising:
(1a) a step of providing the biochip of the present invention, in which a surface treatment with a blocking agent is performed on the substrate, or (1b) a step of allowing a blocking agent to act on the biochip of the present invention, in which a surface treatment with a blocking agent is not performed on the substrate;
(2) a step of allowing a test sample possibly containing a target substance interacting with the material to be immobilized to come into contact with the complex in the biochip; and
(3) a step of allowing a detection reagent to act on the biochip, wherein
   the biochip has a substrate, on the surface of which one or more spots are disposed, each spot comprising a complex of a solid carrier modified with avidin or a derivative thereof and a biotinylated peptide, and wherein
   the detection method has one or more characteristics selected from:
      (1) the peptide is biotinylated via a spacer,
      (2) the biochip has two or more spots, and the volume variation coefficient of the complex among the spots is less than 11.8%,
      (3) the blocking agent comprises biotin or a derivative thereof,
      (4) the detection reagent comprises a monoclonal antibody specific to the target substance, and
      (5) the detection reagent comprises an HRP-labeled antibody specific to the target substance.

In the above-described aspect, the combination of the characteristics is not particularly limited, and for example, it may include the following combinations: (1, 2), (1, 3), (1, 4), (1, 5), (2, 3), (2, 4), (2, 5), (3, 4), (3, 5), (4, 5), (1, 2, 3), (1, 2, 4), (1, 2, 5), (2, 3, 4), (2, 3, 5), (2, 4, 5), (3, 4, 5), (1, 2, 3, 4), (2, 3, 4, 5), and (1, 2, 3, 4, 5). In the aforementioned combinations, for example, (1, 2) means the combination of the characteristic (1) and the characteristic (2). According to the characteristic (1), inhibition of the interaction between a material to be immobilized and a target substance is mainly reduced; according to the characteristic (2), mainly, the uniformity of the reaction strengths of the spots becomes high; according to the characteristic (3), (4) or (5), the SN ratio is mainly improved. Thus, by combining these characteristics with one another, the properties of the detection method can be customized.

In a particularly preferred aspect, the detection method of the present invention comprises:
(1a) a step of providing a biochip having a substrate, on the surface of which two or more spots, each comprising a complex of a solid carrier modified with avidin or a derivative thereof and a peptide biotinylated via a spacer, are disposed, wherein a surface treatment with a blocking agent comprising biotin or a derivative thereof is performed on the substrate, and the volume variation coefficient of the complex among the spots is less than 11.8%, or (1b) a step of allowing a blocking agent comprising biotin or a derivative thereof to act on a biochip having a substrate, on which a surface treatment with a blocking agent is not performed;
(2) a step of allowing a test sample possibly containing a target antibody interacting with the peptide, to come into contact with the complex in the biochip; and
(3) a step of allowing an HRP-labeled monoclonal antibody specific to the target antibody, to act on the biochip.

The present invention also relates to a method of testing the health condition of a subject by using the biochip of the present invention, and a method of testing or diagnosing a disease in a subject by using the biochip of the present invention.

The method of testing the health condition of a subject by using the biochip of the present invention comprises:
(i) a step of providing the above-described biochip comprising a material reacting with a target substance as a material to be immobilized;
(ii) a step of allowing the above-described biochip to react with a biological sample derived from a subject; and
(iii) a step of detecting the reaction of the above-described material to be immobilized with a target substance contained in the biological sample, wherein
   detection or non-detection of the above-described reaction provides information regarding the health condition of the subject.

Examples of the information regarding health condition may include fatigue, stress, and nutritional status. Examples of the target substance may include biological materials regarding the information, such as proteins, nucleic acid molecules, or sugar chains. Examples of the material to be immobilized may include materials reacting with these biological materials, such as antigens, antibodies, antibody mimetics, aptamers, lectins, polynucleotides, nucleic acid mimetics, enzymes, or substrates. Biomarkers used as indicators for fatigue, stress, nutritional status, etc. have been known.

The method of testing or diagnosing a disease in a subject by using the biochip of the present invention comprises:
(i) a step of providing the above-described biochip comprising a material reacting with a target substance as a material to be immobilized;
(ii) a step of allowing the above-described biochip to react with a biological sample derived from a subject; and
(iii) a step of detecting the reaction of the above-described material to be immobilized with a target substance contained in the biological sample, wherein
   detection or non-detection of the above-described reaction provides the presence or absence of the disease in the subject.

The disease may be a disease that can be tested and/or diagnosed by detecting a substance in the biological sample. Specific examples of the disease may include, but are not limited to, allergic disease, endocrine disease, infectious disease, disease with genetic abnormality, inflammatory disease, autoimmune disease, and neoplastic disease. Examples of the target substance may include biological materials regarding such diseases, such as proteins, nucleic acid molecules, or sugar chains. Examples of the material to be immobilized may include materials reacting with these target substances, such as antigens, antibodies, antibody mimetics, aptamers, lectins, polynucleotides, nucleic acid mimetics, enzymes, or substrates. Various types of disease markers such as a tumor marker, an infectious disease marker, a genetic disease marker, an endocrine disease marker, or an inflammation marker have been known.

The biochip of the present invention can be used in each of the above-described intended uses, namely, detection of a target substance, inspection of the health condition of a subject (e.g., fatigue, stress, nutritional status, etc.), and inspection or diagnosis of a disease in a subject (e.g., allergic disease, endocrine disease, infectious disease, disease with genetic abnormality, inflammatory disease, autoimmune disease, neoplastic disease, etc.). On the biochip of the present invention for use in each of the above-described intended uses, a material to be immobilized suitable for each of the above-described intended uses is preferably immobilized.

Another aspect of the present invention also relates to a kit including the above-described biochip, which is for use in the above-described detection, inspection, and diagnosis. The kit of the present invention can be used in each of the above-described intended uses, namely, detection of a target substance, inspection of the health condition of a subject (e.g., fatigue, stress, nutritional status, etc.), and inspection or diagnosis of a disease in a subject (e.g., allergic disease, endocrine disease, infectious disease, disease with genetic abnormality, inflammatory disease, autoimmune disease, neoplastic disease, etc.). The kit of the present invention includes the biochip of the present invention, and preferably the biochip of the present invention comprising a material to be immobilized suitable for each of the above-described intended uses. The kit of the present invention may include a reagent for detecting the reaction of the material to be immobilized with a target substance contained in a biological sample, a blocking agent (in an aspect of using avidin or a derivative thereof as a reactive group, a blocking agent preferably comprising biotin or a derivative thereof), a standard sample, instructions showing the method of using the kit, etc., such as, for example, an instruction manual, information of sites including information regarding use methods (e.g. URL and 2D code), and media that record information regarding use methods (e.g. a flexible disc, CD, DVD, a Blu-ray disc, a memory card, or an USB memory).

Another aspect of the present invention relates to a system for use in carrying out these measurement methods, inspection methods and/or diagnostic methods, wherein the system comprises the biochip of the present invention and a measurement device for a detection reagent.

The measurement device for a detection reagent may be different depending on the detection reagent. Examples of the device may include an absorption spectrometer and an absorption spectrophotometer. The system of the present invention may comprise a user interface, an output device, a communication interface, a controller, a power supply and the like, as well as the aforementioned components.

### Examples

Hereinafter, the present invention will be described in more detail in the following examples. However, these examples are not intended to limit the content of the present invention.

### < Example 1: Production of biochip substrate (1) >

A polycarbonate sheet (manufactured by MITSUBISHI GAS CHEMICAL COMPANY, INC., MU58U, thickness: 0.8 mm) was irradiated with ultraviolet rays, using a UV-ozone irradiation device (manufactured by SEN LIGHTS Co., Ltd., SSP16-110, UV lamp: SUV110GS-36L) at an irradiation distance of 50 mm for 2 minutes. Subsequently, a polyethylene glycol monomethacrylate polymer (manufactured by Sanyu Chemical Co., Ltd.; the molecular weight of a polyethylene glycol moiety: 350) and 4,4'-diazidostilbene-2,2'-disulfonic acid (manufactured by TOKYO CHEMICAL INDUSTRY CO., LTD.; 98%; hereinafter referred to as "bisazide") were each dissolved in a 75% ethanol aqueous solution to result in 0.5 parts by weight and 0.025 parts by weight, respectively, thereby obtaining a coating solution of non-specific adsorption preventing agent. Using a spin-coater (manufactured by MIKASA, MS-A100), the above-described UV-irradiated polycarbonate was coated with 15 µL of this coating solution under conditions of 800 rpm for 5 seconds, and 5000 rpm for 10 seconds. After completion of the coating, using a UV irradiation device (manufactured by UVP, CL-1000L), the substrate was irradiated with ultraviolet rays at 120 mW/cm² for 10 minutes. A silicon rubber-made O ring (14 ϕ) was adhered to the thus coated substrate, using an adhesive, so as to form a reaction area. The aging operation was performed at a temperature of 25°C at a humidity of 60% for 4 to 7 days, so as to obtain a biochip substrate.

### < Example 2: Preparation of stamp solution (1) >

Sodium chloride, potassium chloride, disodium hydrogen phosphate dodecahydrate, and potassium dihydrogen phosphate were each dissolved in ultrapure water to result in 0.8 parts by weight, 0.02 parts by weight, 0.29 parts by weight, and 0.02 parts by weight, respectively, so as to obtain a PBS solution. On the other hand, 4-(2-hydroxyethyl)-1-piperazineethanesulfonic acid was dissolved in ultrapure water to result in 0.59 parts by weight, so as to obtain a HEPES solution. Polyvinyl alcohol (manufactured by Wako Pure Chemical Industries, Ltd., 160-03055) and Tween 20 (manufactured by Sigma-Aldrich, P7949-100ML) were each dissolved in the HEPES solution to result in 0.1 part by weight and 0.05 parts by weight, respectively, so as to obtain a solvent for stamp solution. Biotinylated Peptide 1 formed by directly biotinylating the N-terminus of a peptide selected from full-length αs1-casein, full-length β-casein, full-length β-lactoglobulin and a partial peptide of αs1-casein (consisting of 15 amino acid residues), or Biotinylated Peptide 2 formed by biotinylating the terminus of the above-described peptide via an aminohexyl (Ahx) group (a spacer), were each allowed to react with Streptavidin beads (manufactured by TAMAGAWA SEIKI Co., Ltd.) in the PBS solution at 4°C for 1.5 hours, were then purified, and were then dispersed in the solvent for stamp solution, so as to obtain Stamp Solution 1 (comprising Biotinylated Peptide 1) and Stamp Solution 2 (comprising Biotinylated Peptide 2). Figure 6 shows a schematic view of Biotinylated Peptide 2 immobilized on a bead. In addition, the structures of Biotinylated Peptide 1 and Biotinylated Peptide 2 are shown below.

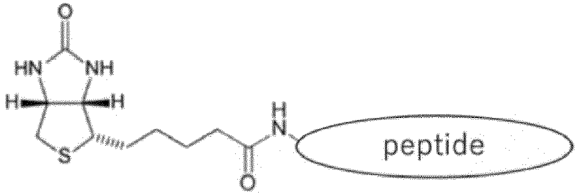

### <Biotinylated Peptide 1>

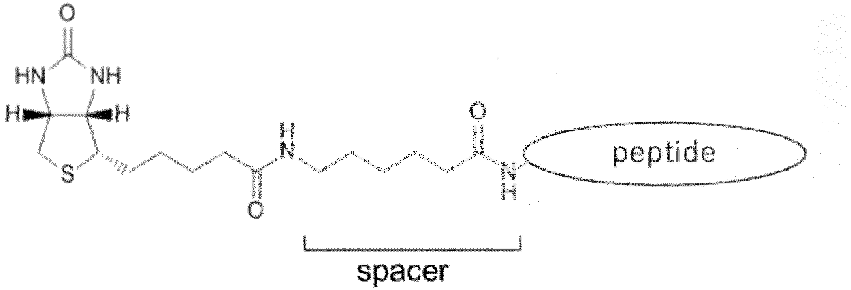

### <Biotinylated Peptide 2>

Besides, there were 20 types of partial peptides, and these partial peptides were each subjected to the above two types of biotinylations. Hereafter, for the sake of convenience, the peptides and the stamp solutions are collectively referred to as "Biotinylated Peptide 1 or 2," and "Stamp Solution 1 or 2," depending on the aspect of biotinylation, regardless of the types of the peptides. Accordingly, for example, Stamp Solution 1 includes Stamp Solution 1A comprising Peptide A, Stamp Solution 1B comprising Peptide B, and the like.

### < Example 3: Production of biochip (1) >

Bisazide was dissolved in ultrapure water to prepare a 10 mg/mL bisazide solution. The 10 mg/mL bisazide solution was diluted by 5 times (Bisazide Solution A), or by 5000 times (Bisazide Solution B). Bisazide Solution B was dissolved in each of Stamp Solutions 1 and 2 to result in 4.8 parts by weight of the Bisazide Solution B.

Using a pin stamper (manufactured by Geneqs, Genex Arrayer) or a non-contact dispenser (manufactured by BioDot, Non-Contact Microdispensing System AD 1520), the thus obtained stamp solutions were each spotted in an amount of 10 nL on the biochip substrate produced in Example 1 according to a lattice point-type multipoint dispensing spot method, to result in 81 spots in total (9 × 9 spots). The diameter of a spot was 0.21 to 0.63 mm (stamper: 214.4 to 397.0 µm, non-contact dispenser: 605.1 to 632.4 µm), and the distance between spots was approximately 1.1 mm. After completion of the spotting, the spots were dried using a vacuum dryer at 0.09 MPa for 10 minutes. After completion of the drying, in order to immobilize beads using the photocrosslinking agent, the spots were irradiated with ultraviolet rays, using a UV irradiation device (manufactured by UVP, CL-1000) at 4 mW/cm² for 10 minutes, so as to obtain Biochips 1 to 3. The peptides and the spotting methods used for individual biochips are shown in the following table.

**Table 1 Outline of individual biochips**

| | Peptide | Spotting method |
|---|---|---|
| Biochip 1 | Biotinylated Peptide 1 | Stamper |
| Biochip 2 | Biotinylated Peptide 1 | Non-contact dispenser |
| Biochip 3 | Biotinylated Peptide 2 | Non-contact dispenser |

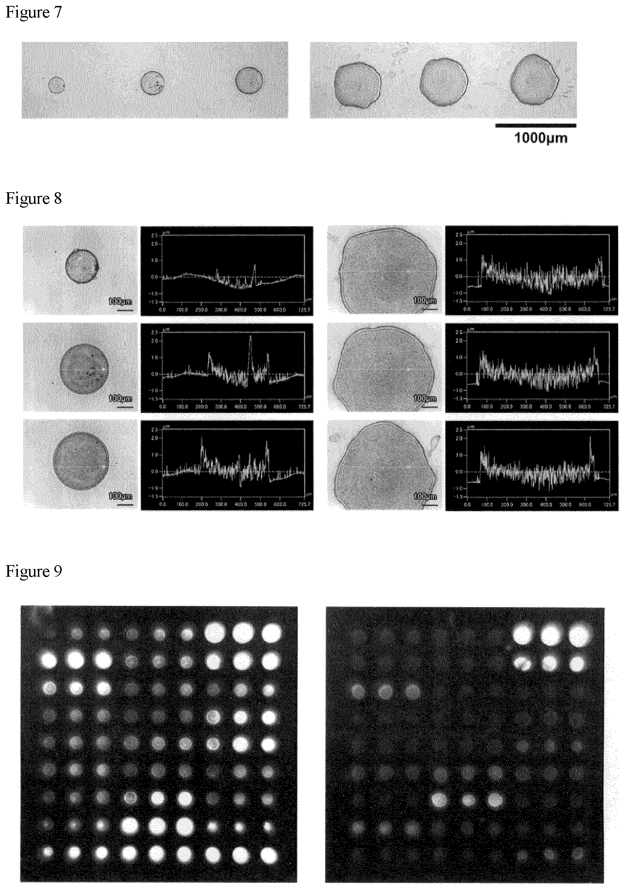

Regarding Biochips 1 and 2, representative spots (3 different types of stamp solutions × 3 spots) were observed with an optical microscope (manufactured by Olympus Corporation, IX71) and with a laser microscope (manufactured by Keyence Corporation, VK-X250). In addition, profile graphs of the representative spots were produced based on 3D images obtained using the laser microscope, and the volume of a solid content present in each spot (spot volume) was then calculated. The results are shown in Figures 7 and 8 and Table 2. In Table 2, the variation coefficient of all spots indicates the variation coefficient of the measured 9 spots as a whole, and the variation coefficient in each stamp solution indicates the variation coefficient range of the spots (n = 3) in each of the three types of stamp solutions. From these results, it became clear that, also in terms of both the size and the volume of a spot, a variation is smaller in the case of using the non-contact dispenser than in the case of using the stamper.

**Table 2 Spot volumes of Biochips 1 and 2**

| | Biochip 1 | Biochip 2 |
|---|---|---|
| Mean ± standard deviation (µm³) | 109102.2 ± 36860.0 | 419785.6 ± 26923.7 |
| Maximum value (µm³) | 158336.0 | 464850.0 |
| Minimum value (µm³) | 28880.0 | 373620.0 |
| Variation coefficient (all spots) (%) | 32.9 | 6.4 |
| Variation coefficient (in each stamp solution) (%) | 11.8 to 49.3 | 2.7 to 3.8 |

### < Example 4: Measurement using chip (1) >

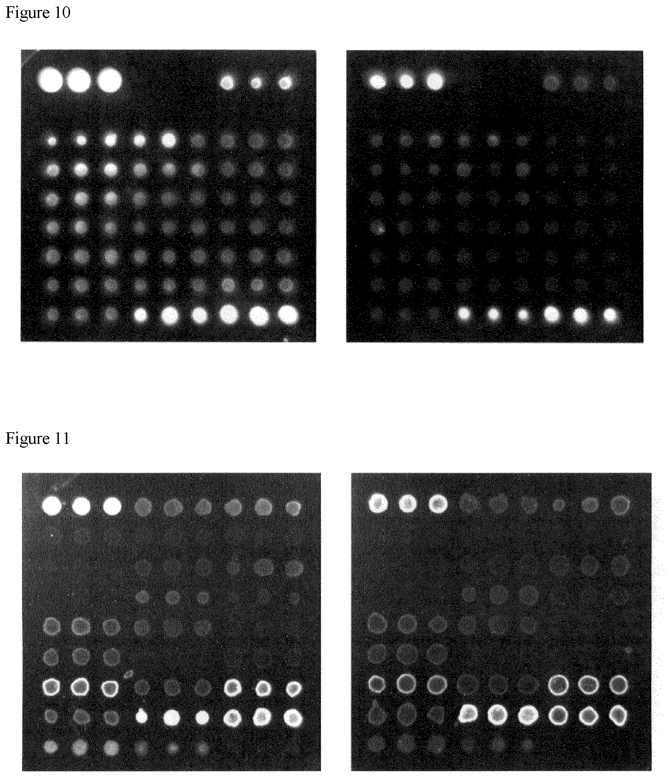

Using the biochip produced in Example 3, an IgE antibody contained in casein-positive human serum (PlasmaLab) was measured. A protein-free blocking agent not containing biotin (Blocking Agent 1, PVDF Blocking Reagent for Can Get Signal (manufactured by Toyobo Co., Ltd., NYPBR01)) or a protein-free blocking agent containing biotin (Blocking Agent 2, PVDF Blocking Reagent for Can Get Signal, to which 0.02% by weight of biotin was added) was added to the reaction area of the biochip, and blocking was then carried out at room temperature (23°C to 27 °C) for 1 hour. Thereafter, the biochip was washed with a TBS-T solution (137 mM sodium chloride, 2.68 mM potassium chloride, 25 mM tris-hydroxymethylaminomethane, pH 7.4, 0.1% by weight of Tween 20) three times. To the reaction area of the biochip, 130 µL of diluted serum was added as a test sample (n = 3 to 5), and while shaking, it was reacted at room temperature for 8 minutes. Besides, 8-fold diluted serum was added to the reaction area to which an ALP-labeled antibody was to be added, and 30-fold diluted serum was added to the reaction area to which an HRP-labeled antibody was to be added. The test sample was aspirated and was then washed with a TBS-T solution. After completion of the washing, 130 µL of a secondary antibody (an ALP-labeled anti-human IgE polyclonal antibody (manufactured by SeraCare Life Sciences, 0751-1004), an ALP-labeled anti-human IgE monoclonal antibody (manufactured by Abcam, ab99805) or an HRP-labeled anti-human IgE monoclonal antibody (manufactured by Abcam, ab99806)) that had been 2000-fold diluted with Can Get Signal Immunoreaction Enhancer Solution 2 (manufactured by Toyobo Co., Ltd., NKB-301) was added to the resultant, and while shaking, the mixture was reacted at room temperature for 4 minutes. Thereafter, the antibody was aspirated and was then washed with TBS-T. To the resultant, 130 µL of a luminescent reagent (Dynalight Substrate with Rapid Glow Enhancer, manufactured by Molecular Probes, to the group on which the ALP-labeled secondary antibody had been allowed to act; whereas Western BLoT Hyper HRP Substrate, manufactured by Takara Bio, T7103A, to the group on which the HRP-labeled secondary antibody had been allowed to act) was added, and the thus obtained mixture was then shaken for 1 minute. Thereafter, using SpotSolver manufactured by Dynacom and Imaged manufactured by NIH, the number of pixels in the luminescent portion was counted, while a portion containing no spot was set as a background, so that the luminescence intensity was measured and the SN ratio was calculated. The outline of experimental groups is shown in Table 3, and the measurement results of the luminescence intensity are shown in Figures 9 to 14 and Tables 4 to 7. Moreover, Table 8 shows the results obtained by performing 5-grade evaluation on individual experimental groups, in terms of the uniformity of luminescence intensity among spots, SN ratio, and a reduction in inhibition of the interaction between a material to be immobilized and a target substance.

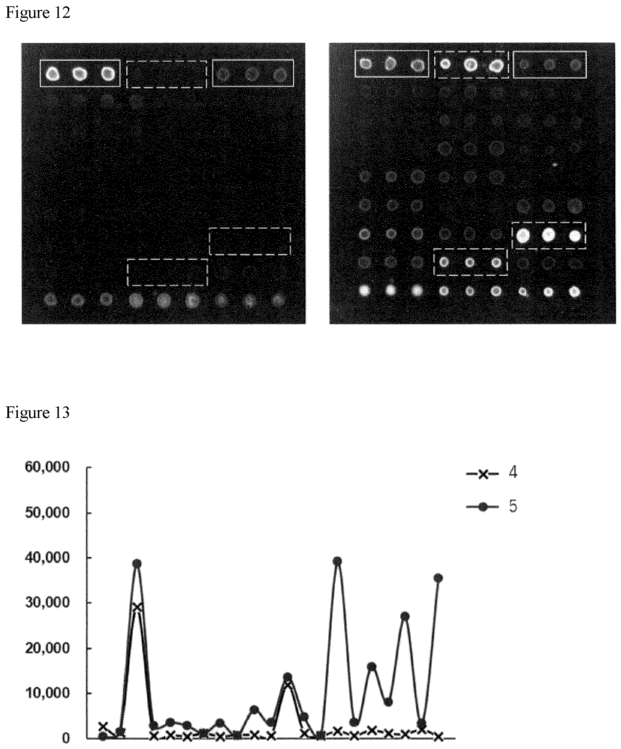

It is to be noted that the uniformity of luminescence intensity among spots was evaluated based on a deviation in the luminescence intensity of multiple spots (n = 3) under identical condition, and that a reduction in inhibition of the interaction was evaluated based on the number of spots that were confirmed to emit luminescence with a luminescence intensity value 1,000 times or more higher than the background value, and the types of materials to be immobilized that were actually immobilized on the spots. For example, in Figure 12, taking into consideration the luminescence status of the spots enclosed with the solid lines in both the left and right photographs, the spots enclosed in the dotted lines in the left photograph are predicted to generally emit luminescence. However, in reality, luminescence was not observed. It is understood that this is not caused by a reduction in the sensitivity of the experimental system as a whole, but is caused by inhibition of the interaction between individual materials to be immobilized and individual target substances (e.g. inhibition of the interaction due to steric hindrance, etc.), although it is not desired to be obsessed with any particular theory. Accordingly, a reduction in inhibition of the interaction between the material to be immobilized and the target substance can be evaluated based on whether or not luminescence is observed in spots in which inhibition of the interaction is likely to take place. As the number of such luminescent spots increases, the degree of a reduction in inhibition of the interaction can be evaluated to be large.

Moreover, there is a case where the types of the spotted peptides are different among the experimental groups. However, the types of the spotted peptides are set to be identical among experimental groups to be subjected to an identical comparative experiment. For instance, in a comparative experiment using Experimental Group 1 and Experimental Group 2, the types of peptides used in both of the experimental groups are identical to each other. On the other hand, the type of a peptide used in Experimental Group 2 in a comparative experiment using Experimental Group 1 and Experimental Group 2 may be different from the type of a peptide used in Experimental Group 2 in a comparative experiment using Experimental Group 2 and Experimental Group 3. However, the effects of the invention demonstrated in each comparative experiment were not influenced by the types of the peptides.

**Table 3 Outline of experimental groups**

| Experimental Group | Spotting method | Spacer | Blocking agent | Label | Type of secondary antibody |
|---|---|---|---|---|---|
| 1 | Stamper | - | Biotin - | ALP | Polyclonal |
| 2 | Non-contact dispenser | - | Biotin - | ALP | Polyclonal |
| 3 | Non-contact dispenser | - | Biotin - | ALP | Monoclonal |
| 4 | Non-contact dispenser | - | Biotin + | ALP | Monoclonal |
| 5 | Non-contact dispenser | + | Biotin + | ALP | Monoclonal |
| 6 | Non-contact dispenser | + | Biotin + | HRP | Monoclonal |

**Table 4 Variation coefficient of luminescence intensity among spots of Experimental Groups 1 and 2**

| | Experimental Group 1 | Experimental Group 2 |
|---|---|---|
| Mean ± standard deviation (%) | 21.4 ± 10.1 | 8.2 ± 5.0 |
| Maximum value (%) | 57.8 | 22.1 |
| Minimum value (%) | 0.7 | 0.3 |

**Table 5 SN ratio of Experimental Groups 2 and 3**

| | Experimental Group 2 | Experimental Group 3 |
|---|---|---|
| Mean ± standard deviation | 17.7 ± 6.3 | 49.8 ± 30.9 |
| Maximum value | 26.7 | 113.3 |
| Minimum value | 6.9 | 12.4 |

**Table 6 SN ratio of Experimental Groups 3 and 4**

| | Experimental Group 3 | Experimental Group 4 |
|---|---|---|
| Mean ± standard deviation | 17.8 ± 6.3 | 32.2 ± 10.9 |
| Maximum value | 27.3 | 44.2 |
| Minimum value | 8.5 | 13.4 |

**Table 7 SN ratio of Experimental Groups 5 and 6**

| | Experimental Group 5 | Experimental Group 6 |
|---|---|---|
| Mean ± standard deviation | 3.6 ± 0.4 | 34.3 ± 12.8 |
| Maximum value | 4.2 | 60.9 |
| Minimum value | 3.2 | 19.0 |

**Table 8 Results of individual experimental groups**

| Experimental Group | Evaluation item (5-grade evaluation, 5: highest evaluation) | | |
|---|---|---|---|
| | Uniformity of luminescence intensity | SN ratio | Reduction in inhibition of interaction |
| 1 | 1 | 1 | 1 |
| 2 | 3 | 1 | 1 |
| 3 | 3 | 2 | 1 |
| 4 | 3 | 3 | 1 |
| 5 | 3 | 3 | 5 |
| 6 | 3 | 4 | 5 |

From the above-described results, it is found that the uniformity of luminescence intensity mainly becomes high by spotting using a non-contact dispenser, that inhibition of the interaction is mainly reduced by using a spacer, and that the SN ratio is mainly improved by the use of a blocking agent comprising biotin, the use of HRP as a label for a secondary antibody, and the use of a monoclonal antibody as a secondary antibody.

### < Example 5: Production of biochip substrate (2) >

### (1) Biochip Substrate A

Biochip Substrate A was obtained in the same manner as that of Example 1.

### (2) Biochip Substrate B

Biochip Substrate B was obtained in the same manner as that of Example 1, with the exceptions that a polystyrene plate (manufactured by Hikari Co., Ltd., PS2032-1, thickness: 1.0 mm) was used instead of the polycarbonate sheet, and that a non-specific adsorbent prepared by dissolving a polymer comprising, as a main component, 2-methacryloyloxy phosphorylcholine (MPC) (manufactured by NOF CORPORATION, LIPIDURE^{®}-CR2001), instead of the polyethylene glycol monomethacrylate polymer, in a 99.5% ethanol solution to result in 0.5 parts by weight of MPC was used as a coating solution.

### (3) Biochip Substrate C

Biochip Substrate C was obtained in the same manner as that of Example 1, with the exceptions that a polyethylene terephthalate film (manufactured by Acrysunday Co., Ltd., Sunday PET, thickness: 1.0 mm) was used instead of the polycarbonate sheet, and that the same coating solution as that for Biochip Substrate B was used.

### (4) Biochip Substrate D

Biochip Substrate D was obtained in the same manner as that of Example 1, with the exceptions that a methyl polymethacrylate plate (manufactured by Asahi Kasei Corporation, Deraglass A) was used instead of the polycarbonate sheet, and that the coating step was not carried out.

### (5) Biochip Substrate E

Biochip Substrate E was obtained in the same manner as that of Example 1, with the exceptions that a polystyrene plate (manufactured by Hikari Co., Ltd., PS2032-1, thickness: 1.0 mm) was used instead of the polycarbonate sheet, and that the coating step was not carried out.

### (6) Biochip Substrate F

Biochip Substrate F was obtained in the same manner as that of Example 1, with the exceptions that a polyethylene terephthalate film (manufactured by Acrysunday Co., Ltd., Sunday PET, thickness: 1.0 mm) was used instead of the polycarbonate sheet, and that the coating step was not carried out.

### < Example 6: Production of biochips and measurement using the same (2) >

Biochips A to F were obtained in the same manner as that of Example 3, with the exceptions that Biochip Substrates A to F produced in Example 5 were used as biochip substrates, that a non-contact dispenser (manufactured by BioDot, Non-Contact Microdispensing System AD1520) was used as a spotting method, and that Stamp Solution 2 was used as a stamp solution.

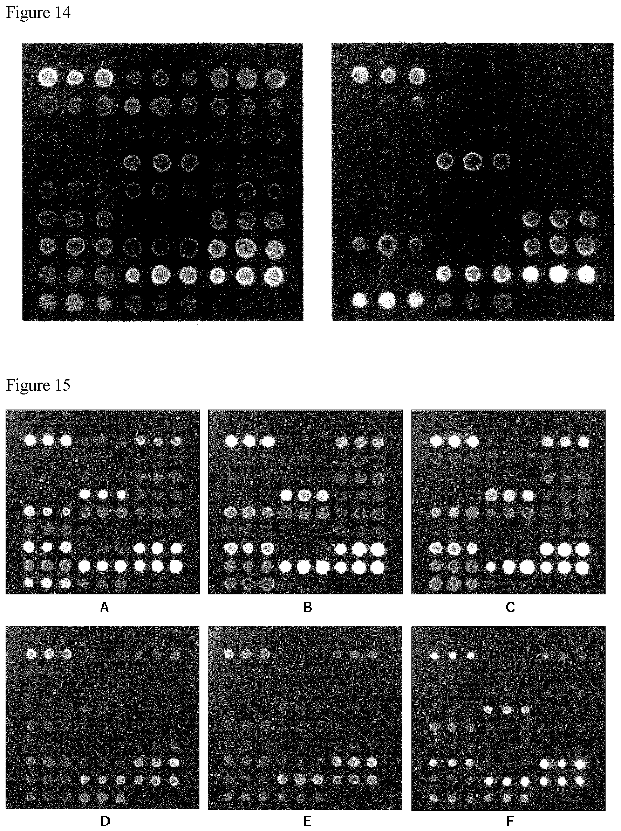

Using the obtained Biochips A to F, the luminescence intensity of each biochip was measured in the same manner as that of Example 4, with the exceptions that Blocking Agent 2 was used as a blocking agent, and that an ALP-labeled anti-human IgE monoclonal antibody (manufactured by Abcam, ab99805) was used as a secondary antibody. The results are shown in Figure 15. From all of the Biochips A to F, good measurement results were obtained, as with Experimental Group 5 of Example 4. Moreover, better results were obtained from the case of using a substrate having a coating layer, than the case of using a substrate not having such a coating layer.

### Reference Signs List

10: Biochip (A)
11: Biochip (B)
12: Biochip (C)
13: Biochip (D)
101: Substrate
101A: Upper surface of substrate
102: Boundary
103: Reaction area
104: Coating layer
105: Spot

## Claims

1. A biochip having a substrate, wherein one or more spots are disposed on a surface of the substrate, each spot comprising a complex of a solid carrier modified with a reactive group and a material to be immobilized modified with a group reacting with the reactive group via a spacer.

2. The biochip according to claim 1, wherein the reactive group is selected from the group consisting of avidin or a derivative thereof, biotin or a derivative thereof, alkyne, and azide.

3. The biochip according to claim 1 or 2, wherein two or more spots are disposed, and the volume variation coefficient of the complex comprised in a single spot is less than 11.8%.

4. The biochip according to any one of claims 1 to 3, wherein a surface treatment with a blocking agent is performed on the substrate.

5. A method of producing a biochip, comprising spotting on a substrate a complex of a solid carrier modified with a reactive group and a material to be immobilized modified with a group reacting with the reactive group via a spacer, and immobilizing the spotted complex on the substrate.

6. The method of producing a biochip according to claim 5, wherein spotting the complex on the substrate is carried out by using a non-contact dispenser comprising a nozzle with a diameter that is 100 to 2000 times greater than the average maximum diameter of the solid carrier.

7. A material set for use in producing the biochip according to any one of claims 1 to 4, comprising a solid carrier modified with a reactive group, a material to be immobilized modified with a group reacting with the reactive group via a spacer, and a substrate.

8. A method of detecting a target substance, comprising:
providing the biochip according to claim 4, or allowing a blocking agent to act on the biochip according to any one of claims 1 to 3, in which a surface treatment with a blocking agent is not performed on the substrate;
allowing a test sample possibly containing a target substance interacting with the material to be immobilized to come into contact with the complex in the biochip; and
allowing a detection reagent to act on the biochip.

9. The detection method according to claim 8, wherein the reactive group is avidin or a derivative thereof, and the blocking agent is biotin or a derivative thereof.

10. The detection method according to claim 8 or 9, wherein the target substance is an antibody, and the detection reagent comprises a monoclonal antibody specific to the antibody.

11. The detection method according to any one of claims 8 to 10, wherein the target substance is an antibody, and the detection reagent comprises an HRP-labeled antibody specific to the antibody.

12. A kit for use in carrying out the detection method according to any one of claims 8 to 11, wherein the kit comprises the biochip according to any one of claims 1 to 4, a detection reagent, and as necessary, a blocking agent.

13. A system for use in carrying out the detection method according to any one of claims 8 to 11, wherein the system comprises the biochip according to any one of claims 1 to 4 and a measurement device for a detection reagent.
